# EUROPÄISCHE PATENTSCHRIFT

(11) **EP 3 763 325 B1**
(45) Veröffentlichungstag und Bekanntmachung des Hinweises auf die Patenterteilung: **06.10.2021**
(21) Anmeldenummer: 19185658.2
(22) Anmeldetag: 11.07.2019
(51) Int. Cl.: A61C 19/02

(54) **TRÄGERELEMENT ZUM LAGERN ZUMINDEST EINES MEDIZINISCHEN ODER DENTALEN INSTRUMENTS IN EINEM REINIGUNGS- ODER PFLEGEGERÄT**
CASSETTE FOR STORING AT LEAST ONE MEDICAL OR DENTAL INSTRUMENT IN A CLEANING OR CARE DEVICE
CASSETTE DESTINÉE À STOCKER AU MOINS UN INSTRUMENT MÉDICAL OU DENTAIRE DANS UN APPAREIL DE NETTOYAGE OU DE SOIN

(43) Veröffentlichungstag der Anmeldung: 13.01.2021
(73) Patentinhaber: W & H Dentalwerk Bürmoos GmbH, 5111 Bürmoos (AT)
(72) Erfinder: PRUCKNER, Christian, 1190 Wien (AT); KAINHOFER, Manfred, 5113 St. Georgen (AT); REITER, Michael, 5061 Elsbethen (AT)
(74) Vertreter: Benda, Ralf

(56) Entgegenhaltungen:
- WO-A1-2019/012181
- JP-A- 2016 507 259
- US-A1- 2007 160 494
- US-A1- 2008 296 373
- US-A1- 2018 153 639

## Beschreibung

Die vorliegende Erfindung betrifft ein Trägerelement zum Lagern zumindest eines medizinischen oder dentalen Instruments in einem Reinigungs- oder Pflegegerät sowie ein Verfahren zur drahtlosen Übertragung von Daten und/ oder Energie aufweisender elektromagnetischer Strahlung mit Hilfe eines derartigen Trägerelements.

Aus der Anmeldeschrift US 2014/0212833 A1 ist eine Kupplungsvorrichtung zur drahtlosen Übertragung von Daten und/ oder Energie zwischen einem medizinischen, insbesondere dentalen, Instrument und einem damit verbindbaren Kupplungsteil bekannt. Dieses Kupplungsteil kann auch im Inneren, zum Beispiel in einer Kammer, eines Reinigungs- oder Pflegegeräts angeordnet sein, so dass Daten und/ oder Energie drahtlos zwischen einem medizinischen, insbesondere dentalen, Instrument und dem Reinigungs-oder Pflegegerät übertragbar sind.

Die Übertragung von Daten und/ oder Energie an einem Reinigungs- oder Pflegegerät, insbesondere innerhalb des Reinigungs- oder Pflegegerät oder aus dem Reinigungs- oder Pflegegerät heraus, ist unter anderem aufgrund metallischer Bauteile des Reinigungs- oder Pflegegeräts oder während des Betriebs vorhandener elektromagnetischer Felder herausfordernd. Dadurch entstehen Probleme wie eine verringerte ÜbertragungsReichweite oder Beeinflussung und Störung der Übertragung von Daten und/ oder Energie.

Die aus der im Vorstehenden genannten Anmeldeschrift US'833 A1 bekannte Lösung umgeht diese Probleme durch die Anordnung der Sende- und Empfangseinheiten des Instruments und des Reinigungs- oder Pflegegeräts an den jeweiligen Kupplungen, so dass die beiden Sende- und Empfangseinheiten möglichst nahe aneinander positioniert sind. Diese Lösung funktioniert grundsätzlich gut, jedoch ist dadurch die Anzahl der Instrumente, die gereinigt oder gepflegt und mit denen Daten und/ oder Energie ausgetauscht werden können, auf die Anzahl der vorhandenen Kupplungsteile des Reinigungs- oder Pflegegeräts beschränkt.

Aus der Anmeldeschrift WO 2016/188959 A1 ist ein Trägerelement bekannt, in dem sich eine mattenförmige Einlage befindet. Auf dieser Einlage ist eine Erfassungsvorrichtung mit einem Sensor und einem Funkmodul, insbesondere eine RFID-Leseeinrichtung, vorgesehen, die ausgebildet ist, Daten aus einem Speicher eines in dem Trägerelement aufgenommenen Instruments auszulesen und drahtlos an ein Endgerät zu übertragen.

In entsprechender Weise beschreibt die Anmeldeschrift US 2008/0296373 A1 ein Trägerelement mit einem aktiven Transponderetikett, das passive Etiketten von Instrumenten ausliest und die ausgelesenen Daten an ein Informationssystem drahtlos weiterleitet.

Der vorliegenden Erfindung liegt daher die Aufgabe zugrunde eine Möglichkeit zu schaffen, eine gesicherte Daten- und/ oder Energieübertragung für zu reinigenden oder zu pflegende medizinische, insbesondere dentale, Instrument, die sich nahe an oder in einem Reinigungs- oder Pflegegeräts befinden, zu ermöglichen. Die Daten- und/ oder Energieübertragung soll dabei insbesondere auch ohne Anschluss des medizinischen, insbesondere dentalen, Instruments an ein Kupplungsteil des Reinigungs- oder Pflegegeräts zuverlässig und sicher sein.

Diese Aufgabe wird gemäß der vorliegenden Erfindung durch ein Trägerelement zum Lagern zumindest eines medizinischen oder dentalen Instruments in einem Reinigungs- oder Pflegegerät mit den Merkmalen des Anspruchs 1, durch ein Reinigungs- oder Pflegegerät mit den Merkmalen des Anspruchs 11, durch eine Verwendung eines erfindungsgemäßen Trägerelements mit den Merkmalen des Anspruchs 12 und durch ein Verfahren zur drahtlosen Übertragung von Daten und/ oder Energie mit den Merkmalen des Anspruchs 13 gelöst. Besonders vorteilhafte Ausführungsformen sind in den jeweiligen Unteransprüchen angeführt.

Das Trägerelement ist ausgebildet, zumindest ein medizinisches oder dentales Instrument in einem Reinigungs- oder Pflegegerät zu lagern oder zu halten, um das zumindest eine medizinische oder dentale Instrument in dem Reinigungs- oder Pflegegerät zu reinigen oder zu pflegen. An dem medizinischen oder dentalen Instrument ist eine erste Sende- und Empfangseinheit angeordnet, die zur drahtlosen Übertragung von Daten und/ oder Energie aufweisender elektromagnetischer Strahlung mit einer zweiten Sende- und Empfangseinheit ausgebildet ist. An dem Trägerelement ist zumindest eine Koppelantenne vorgesehen, die ausgebildet ist, die drahtlos zwischen der ersten Sende- und Empfangseinheit und der zweiten Sende- und Empfangseinheit übertragene elektromagnetische Strahlung zu empfangen und wiederum abzustrahlen, um die drahtlose Übertragung der Daten und/ oder Energie aufweisenden elektromagnetischen Strahlung zwischen der ersten Sende- und Empfangseinheit und der zweiten Sende- und Empfangseinheit zu unterstützen.

Soweit nicht anders erwähnt gilt für alle beschriebenen Ausführungsbeispiele, dass die drahtlose Übertragung der Daten und/ oder Energie aufweisenden elektromagnetischen Strahlung zwischen der ersten Sende- und Empfangseinheit und der zweiten Sende- und Empfangseinheit eine Übertragung in Richtung der der ersten Sende- und Empfangseinheit und/ oder in Richtung der zweiten Sende- und Empfangseinheit umfasst. Die erste und zweite Sende- und Empfangseinheit sind vorzugsweise zur Daten- und/ oder Energieübertragung im UHF- und/ oder SHF-Frequenzbereich ausgebildet. Die erste Sende-und Empfangseinheit des medizinischen oder dentalen Instruments umfasst zum Beispiel ein RFID-Etikett. Die zweite Sende- und Empfangseinheit umfasst zum Beispiel ein Lesegerät zum Auslesen der ersten Sende- und Empfangseinheit.

Das Vorsehen der zumindest einen Koppelantenne an dem Trägerelement verbessert und unterstützt in vorteilhafter Weise die Daten- und/ oder Energieübertragung zwischen der ersten Sende- und Empfangseinheit und der zweiten Sende- und Empfangseinheit, vorzugsweise im Inneren eines Reinigungs- oder Pflegegeräts oder beim Einbringen des Trägerelements in das Reinigungs- oder Pflegegerät oder beim Entnehmen des Trägerelements aus dem Reinigungs- oder Pflegegerät, so dass es insbesondere nicht mehr nötig ist zur Daten- und/ oder Energieübertragung ein medizinisches, insbesondere dentales, Instrument an ein Kupplungsteil des Reinigungs- oder Pflegegeräts anzuschließen.

Die zumindest eine Koppelantenne ist insbesondere dazu ausgebildet und vorgesehen, dem im Vorstehenden genannten negativen Einfluss der metallischen Bauteile des Reinigungs- oder Pflegegeräts und der elektromagnetischen Felder auf die Übertragung der elektromagnetischen Strahlung entgegenzuwirken oder diesen Einfluss zu verringern. Die zumindest eine Koppelantenne ist besonders bevorzugt vorgesehen, die erste Sende-und Empfangseinheit und die zweite Sende- und Empfangseinheit kommunikativ miteinander zu koppeln oder zu verbinden.

Die Unterstützung und/ oder Verbesserung der drahtlosen Übertragung der Daten und/ oder Energie aufweisenden elektromagnetischen Strahlung durch die zumindest eine Koppelantenne umfasst beispielsweise:
- eine Beeinflussung oder Änderung der Abstrahlrichtung der Daten und/ oder Energie aufweisenden elektromagnetischen Strahlung, insbesondere in Richtung der ersten Sende- und Empfangseinheit und/ oder der zweiten Sende- und Empfangseinheit;
- eine Erhöhung der Energiedichte der Daten und/ oder Energie aufweisenden elektromagnetischen Strahlung;
- eine Bündelung oder Fokussierung der Daten und/ oder Energie aufweisenden elektromagnetischen Strahlung;
- eine Erhöhung der Übertragungsreichweite der Daten und/ oder Energie aufweisenden elektromagnetischen Strahlung, insbesondere aufgrund der im Vorstehenden genannten Beeinflussung oder Änderung der Abstrahlrichtung, Erhöhung der Energiedichte und/ oder Bündelung oder Fokussierung;
- eine Verringerung des Verlustes der übertragenen elektromagnetischen Strahlung, insbesondere der Daten und/ oder Energie, insbesondere aufgrund der im Vorstehenden genannten Beeinflussung oder Änderung der Abstrahlrichtung, Erhöhung der Energiedichte und/ oder Bündelung oder Fokussierung.

Das Trägerelement umfasst zum Beispiel eine Trägerplatte, ein Tray, einen Korb oder eine Kassette oder ist als eines dieser genannten Elemente ausgebildet.

Die Trägerplatte ist insbesondere als Platte oder Gitterplatte ohne Seitenwände und ohne Abdeckung oder Deckel ausgebildet. Vorzugsweise ist die Trägerplatte als im Wesentlichen ebene Fläche ausgebildet. Vorzugsweise ist der Außenumfang der Trägerplatte durch einen Rahmen mit mehreren gewinkelt zueinander angeordneten Seiten ausgebildet. Vorzugsweise umgibt der Rahmen die Platte oder das Gitter, welche(s) eine Vielzahl von Öffnungen oder Gitteröffnungen aufweist. Vorzugsweise sind die Öffnungen oder Gitteröffnungen als Durchlass für ein Reinigungs- oder Pflegemittel vorgesehen. Vorzugsweise umfasst der Rahmen keine Öffnungen oder Gitteröffnungen. Vorzugsweise ist die zumindest eine Koppelantenne an oder in der Trägerplatte, insbesondere der Gitterplatte und/ oder dem Rahmen, vorgesehen.

Der Tray oder Korb umfasst insbesondere eine Grundplatte oder einen Boden und mehrere damit verbundene und die Grundplatte oder den Boden umgebende Seitenwände. Vorzugsweise umfasst/ umfassen der Boden und/ oder die Seitenwände eine Vielzahl von Öffnungen. Vorzugsweise sind der Tray oder Korb als Sieb oder Siebkorb mit einer Vielzahl von Sieböffnungen ausgebildet. Vorzugsweise sind die Öffnungen oder Sieböffnungen als Durchlass für ein Reinigungs- oder Pflegemittel vorgesehen. Vorzugsweise ist die zumindest eine Koppelantenne an oder in der Grundplatte oder dem Boden und/ oder an oder in zumindest einer der Seitenwände vorgesehen.

Die Kassette umfasst insbesondere einen Boden, mehrere damit verbundene und den Boden umgebende Seitenwände und einen Deckel oder eine Abdeckung. Vorzugsweise ist der Deckel oder die Abdeckung relativ zu dem Boden und/ oder den Seitenwänden beweglich, zum Beispiel verschwenkbar, oder davon entfernbar ausgebildet. Vorzugsweise überdeckt der Deckel oder die Abdeckung die mehreren Seitenwände. Vorzugsweise grenzen der Boden, die Seitenwände und der Deckel oder die Abdeckung einen Innenraum der Kassette gegenüber der Umgebung ab. Vorzugsweise sind an dem Boden und/ oder an den Seitenwänden und/ oder an dem Deckel oder der Abdeckung eine Vielzahl von Öffnungen, zum Beispiel längliche Öffnungen, vorgesehen, die insbesondere als Durchlass für ein Reinigungs- oder Pflegemittel dienen. Vorzugsweise ist die zumindest eine Koppelantenne an oder in dem Boden und/ oder an oder in zumindest einer der Seitenwände und/ oder an oder in dem Deckel oder der Abdeckung vorgesehen.

Das Trägerelement ist insbesondere dazu ausgebildet, in einem Reinigungs- oder Pflegegerät aufgenommen zu werden. Das Trägerelement ist zum Beispiel dazu ausgebildet, auf die Innenseite eines Bodens des Reinigungs- oder Pflegegerät gestellt zu werden oder in eine Kammer des Reinigungs- oder Pflegegeräts eingebracht zu werden. Das Trägerelement ist vorzugweise dazu ausgebildet, an einem oder mehreren Halteelementen des Reinigungs-oder Pflegegeräts gelagert oder lösbar befestigt zu werden. Das Halteelement umfasst zum Beispiel eine Schiene oder Leiste oder einen Vorsprung, an dem das Trägerelement, insbesondere ein zum Halteelement passendes oder komplementäres oder damit verbindbares Lagerelement des Trägerelements, lagerbar oder befestigbar ist. Das Halteelemente des Reinigungs- oder Pflegegeräts ist insbesondere an der Innenseite der Kammer vorgesehen. Das Lagerelement des Trägerelements ist insbesondere an dessen Boden und/ oder an zumindest einer der mehreren Seitenwände vorgesehen.

Das Trägerelement ist vorzugsweise zumindest teilweise aus Metall und/ oder Kunststoff und/ oder Glas und/ oder Keramik gefertigt. Das Metall umfasst zum Beispiel Stahl oder eine Stahllegierung. Die Auswahl des Materials des Trägerelements, insbesondere jenes Teils des Trägerelements an dem die zumindest eine Koppelantenne vorgesehen oder angeordnet ist, ist vorzugsweise von der Art der Koppelantenne abhängig. So ist es insbesondere für die Funktion einer als Flächenantenne, insbesondere als Schlitzantenne, ausgebildeten Koppelantenne vorteilhaft, wenn diese von einem metallischen Bauteil des Trägerelements umgeben ist. Das metallische Bauteil kann zum Beispiel ein den Schlitz oder die Schlitzantenne umgebender metallischer Rahmen oder eine metallische Beschichtung eines den Schlitz umgebenden Teils des Trägerelements sein, zum Beispiel eines Teils des Bodens, zumindest einer Seitenwand oder der Abdeckung. Das metallische Bauteil kann auch ein den Schlitz umgebendes, metallisches Teil des Trägerelements umfassen, zum Beispiel zumindest einen metallischen Teil des Bodens, zumindest einer Seitenwand oder der Abdeckung. Für die Funktion einer als metallische, (geometrisch) lineare Antenne, zum Beispiel als Stabantenne, ausgebildeten Koppelantenne ist es von Vorteil, wenn diese von einem elektrisch nichtleitenden Material, zum Beispiel Glas oder Kunststoff, umgeben ist. Demgemäß ist vorzugsweise ein Abschnitt des Trägerelements, zum Beispiel ein Teil des Bodens, zumindest einer Seitenwand oder der Abdeckung, an oder in dem die (geometrisch) lineare Antenne angeordnet ist, aus einem elektrisch nichtleitenden Material gebildet.

Vorzugsweise umfasst die an dem medizinischen oder dentalen Instrument angeordnete erste Sende- und Empfangseinheit oder Transpondereinheit, insbesondere das RFID-Etikett, eine Antenne, insbesondere einen Schwingkreis, zum Empfangen und/ oder Senden der Daten und/ oder Energie aufweisenden elektromagnetischen Strahlung. Die erste Sende- und Empfangseinheit umfasst des Weiteren insbesondere einen Kondensator und/ oder ein Speicherelement, die operativ mit der Antenne verbunden sind. Auf dem Speicherelement sind insbesondere Daten zur Identifizierung des Instruments gespeichert, zum Beispiel ein dem Instrument, an dem die erste Sende- und Empfangseinheit befestigt ist, (eindeutig) zuordenbarer Identifizierungscode oder Codes zur Identifizierung des Instruments über eine externe Datenbank, zum Beispiel nach GS1 oder HIBC-Standard.

Vorzugsweise ist die erste Sende- und Empfangseinheit als passive erste Sende- und Empfangseinheit ausgebildet, die insbesondere für die Kommunikation und für interne Prozesse benötigte Energie ausschließlich aus der elektromagnetischen Strahlung erhält, die von der zweiten Sende-/Empfangseinheit abgegeben wird. Besonders bevorzugt umfasst die erste Sende- und Empfangseinheit ein oder mehrere RFID-Etikette.

Die von der ersten Sende- und Empfangseinheit entfernt angeordnete zweite Sende-und Empfangseinheit umfasst insbesondere eine Lesevorrichtung zum Auslesen und/ oder Empfangen der in dem Speicherelement der ersten Sende- und Empfangseinheit gespeicherten instrumentenspezifische Daten, insbesondere des Identifizierungscodes. Vorzugsweise umfasst die zweite Sende- und Empfangseinheit wiederum eine Antenne, insbesondere einen Schwingkreis, zum Empfangen und/ oder Senden der elektromagnetischen Strahlung, eine Quelle für die elektromagnetische Strahlung, zum Beispiel ein Hochfrequenzmodul, und einen Controller. Vorzugsweise umfasst die zweite Sende- und Empfangseinheit auch eine Schnittstelle zu einer Verarbeitungseinheit, um die empfangenen Daten weiter zu verarbeiten. Die von der zweiten Sende- und Empfangseinheit emittierte elektromagnetische Strahlung liefert der ersten Sende- und Empfangseinheit Energie für den Betrieb. Die zweite Sende- und Empfangseinheit ist entweder an oder in dem Reinigungs- oder Pflegegerät befestigt oder räumlich davon getrennt.

Wenn die erste und zweite Sende- und Empfangseinheit kommunikativ miteinander verbunden sind, d.h. wenn die erste Sende- und Empfangseinheit sich im Lesebereich der zweiten Sende- und Empfangseinheit oder sich in einem von der zweiten Sende- und Empfangseinheit ausgestrahlten elektromagnetischen Feld befindet, insbesondere wenn das medizinische oder dentale Instrument in dem Reinigungs- oder Pflegegerät, zum Beispiel in einer Kammer des Reinigungs- oder Pflegegeräts, aufgenommen wird oder ist, ist die zweite Sende- und Empfangseinheit ausgebildet, die instrumentenspezifischen Daten, insbesondere den Identifizierungscode, durch die drahtlose Übertragung der Daten und/ oder Energie aufweisenden elektromagnetischen Strahlung zu bestimmen oder zu erkennen. Vorzugsweise werden die derart bestimmten instrumentenspezifischen Daten an eine Verarbeitungseinheit, zum Beispiel einen Prozessor oder Mikrocontroller, der mit der zweiten Sende- und Empfangseinheit kommunikativ verbunden ist, weitergeleitet. Vorzugsweise ist die Verarbeitungseinheit ausgebildet, diese weitergeleiteten instrumentenspezifischen Daten zu speichern und/ oder zu verarbeiten, um insbesondere nachzuweisen, dass das medizinische oder dentale Instrument, dessen instrumentenspezifische Daten, insbesondere dessen Identifizierungscode, empfangen und weitergeleitet wurden, in dem Reinigungs- oder Pflegegerät aufgenommen und/ oder gereinigt oder gepflegt wurde.

Das medizinische oder dentale Instrument, an dem die erste Sende- und Empfangseinheit angeordnet ist, umfasst zum Beispiel: ein Instrument zur medizinischen oder dentalen Diagnose; ein Instrument zur medizinischen oder dentalen Behandlung; ein Instrument zur Abgabe eines Mediums und/ oder Energie auf eine Diagnose- oder Behandlungsstelle; ein Instrument zum rotierenden oder oszillierenden oder schwingenden Antrieb eines Werkzeugs; eine Antriebsvorrichtung, zum Beispiel einen Motor; einen Adapter; eine Kupplungsvorrichtung; ein Verbindungsstück; ein aktives Instrument, das zum Betrieb die Zufuhr von Energie von einer Energiequelle benötigt; ein passives Instrument ohne Zufuhr von Energie, zum Beispiel ein Schaber, eine Pinzette oder eine Schere; ein Handstück, Handgriffelement oder Winkelstück; ein Werkzeug, zum Beispiel ein Bohrer, eine Klinge, ein Sägeblatt, eine Scalerspitze, ein Shaver, eine Düse, eine Licht oder Laser abgebende Spitze, und ähnliche Werkzeuge.

Vorzugsweise ist an dem Trägerelement zumindest ein mechanisches Positionierelement, zum Beispiel eine Halterung, vorgesehen, um das zumindest eine medizinische oder dentale Instrument an dem Trägerelement zu positionieren, insbesondere zu fixieren. Besonders bevorzugt ist das Positionierelement derart ausgebildet, dass das medizinische oder dentale Instrument in einer (vor)bestimmten Position, Orientierung und/ oder Anordnung in Bezug auf das Trägerelement und/ oder die zumindest eine Koppelantenne angeordnet ist, so dass die erste Sende- und Empfangseinheit zu der zumindest einen Koppelantenne gewandt ist und/ oder sich in Richtung der zumindest einen Koppelantenne erstreckt. Beispielsweise ist das Positionierelement oder die Halterung derart bemessen oder geformt, dass nur ein bestimmter Abschnitt des medizinischen oder dentalen Instruments darin aufnehmbar ist, wodurch die vorbestimmte Position, Anordnung oder Orientierung eingenommen ist. Alternativ oder zusätzlich sind an dem medizinischen oder dentalen Instrument und an dem Positionierelement jeweils zumindest ein mechanisches Eingriffselement vorgesehen, so dass, wenn die Eingriffselemente ineinandergreifen oder einrasten, die vorbestimmte Position, Anordnung oder Orientierung eingenommen ist. Durch derartige Positionierelemente wird in vorteilhafter Weise die drahtlose Kommunikation, insbesondere die Übertragung der elektromagnetischen Strahlung, zwischen der ersten Sende- und Empfangseinheit und der zumindest einen Koppelantenne verbessert.

Vorzugsweise weist das zumindest eine Positionierelement ein Steckelement mit einer Steckaufnahme auf, in welche das zu reinigende oder pflegende medizinische oder dentale Instrument einsteckbar ist. Alternativ oder zusätzlich weist das zumindest eine Positionierelement ein Klemmelement auf oder ist die Steckaufnahme als Klemmelement ausgebildet, in welches das zu reinigende oder pflegende medizinische oder dentale Instrument klemmbar ist. Vorzugsweise definiert die Steckaufnahme oder das Klemmelement einen Aufnahmeraum, in dem zumindest ein Teil des medizinische oder dentale Instruments aufnehmbar, insbesondere klemmbar ist. Besonders bevorzugt ist eine den Aufnahmeraum begrenzende Wand gebogen oder kreissegmentförmig geformt. Besonders bevorzugt ist der Aufnahmeraum oder die ihn begrenzende Wand derart bemessen oder geformt oder weist eines der Eingriffselemente auf, um das medizinische oder dentale Instrument in der (vor)bestimmten Position zu positionieren, so wie dies im Vorstehenden beschrieben ist. Vorzugsweise weist die Steckaufnahme oder das Klemmelement zwei Federarme oder zwei Lagerarme auf, zwischen denen der Aufnahmeraum vorgesehen ist. Die zwei Federarme oder Lagerarme entspringen vorzugsweise einer gemeinsamen Basis. Die zwei Federarme oder Lagerarme sind vorzugsweise gebogen geformt. Die zwei Federarme oder Lagerarme weisen bevorzugt jeweils ein freies Ende auf.

Vorzugsweise ist das zumindest eine Positionierelement einteilig mit Trägerelement ausgebildet, zum Beispiel am Boden, an der Grund- oder Gitterplatte und/ oder an einer der Seitenwände vorgesehen. Alternativ ist das zumindest eine Positionierelement lösbar an dem Trägerelement befestigt. Vorzugsweise sind dazu mehrere kleine Öffnungen, zum Beispiel im Wesentlichen kreisrunde Öffnungen an dem Trägerelement vorgesehen, in welche ein Verbindungselement, zum Beispiel ein Fortsatz, des zumindest einen Positionierelements einsteckbar ist, um das zumindest eine Positionierelement an dem Trägerelement zu befestigen. Wenn das Trägerelement ein Gitter oder eine Gitterplatte aufweist, so wie dies im Vorstehenden schon beschrieben ist, so ist es auch möglich, das zumindest eine Positionierelement an dem Gitter oder der Gitterplatte, insbesondere durch Einstecken des Fortsatzes des Trägerelements in eine oder mehreren Gitteröffnungen, zu befestigen.

Die zumindest eine Koppelantenne ist vorzugsweise als Teil des Trägerelements ausgebildet, insbesondere unlösbar mit dem Trägerelement verbunden und/ oder integral mit dem Trägerelement ausgebildet, insbesondere als Flächen- oder Schlitzantenne. Alternativ ist es auch möglich, die zumindest eine Koppelantenne als separates Bauteil auszubilden, das in das Trägerelement eingelegt und/ oder mit dem Trägerelement verbunden ist und insbesondere auch wieder daraus entfernbar ist. Somit kann in vorteilhafter Weise ein Nachrüsten eines Trägerelements verwirklicht werden. Vorzugsweise ist ein Haltekörper vorgesehen, zum Beispiel eine Platte, an oder in dem die zumindest eine Koppelantenne vorgesehen ist, zum Beispiel in Form eines Schlitzes in dem Haltekörper, um eine Schlitzantenne zu bilden. Alternativ ist an oder in dem Haltekörper eine (geometrisch) lineare Antenne ausgebildet, zum Beispiel in Form einer Stab- oder Drahtantenne, so wie dies im Folgenden beschrieben ist.

Vorzugsweise ist die zumindest eine Koppelantenne als (geometrisch) lineare Antenne ausgebildet, deren elektrischer Leiter insbesondere einen metallischen Draht oder einen metallischen Stab aufweist. Die (geometrisch) lineare Antenne ist insbesondere als Stabantenne oder als Drahtantenne oder als Dipolantenne ausgebildet. Durch die Ausbildung der Koppelantenne als lineare Antenne ist es in vorteilhafter Weise möglich, die Abmessung, insbesondere die Länge der Antenne unabhängig von den Abmessungen des Trägerelements zu wählen, insbesondere ist es möglich, dass die Länge der Antenne größer ist als die längste Abmessung oder die längste Außenseite des Trägerelements.

Vorzugsweise ist die (geometrisch) lineare Antenne in einer Ausnehmung des Trägerelements angeordnet, insbesondere in einem Durchbruch einer Außenwand des Trägerelements, zum Beispiel des Bodens oder der Bodenplatte, einer Seitenwand oder der Abdeckung. Dadurch ist die lineare Antenne in vorteilhafter Weise in das Trägerelement, insbesondere in dessen Körper integriert, wodurch die Handhabung des Trägerelements vereinfacht ist. Um eine noch bessere Integration zu bewirken, ist die Ausnehmung, in der die lineare Antenne angeordnet ist, bevorzugt mit einem elektrisch nichtleitenden Füllmaterial gefüllt, zum Beispiel Kunststoff oder Glas oder Keramik. Bevorzugt ist die lineare Antenne von einem elektrisch nichtleitenden Material umgeben, zum Beispiel Kunststoff oder Glas oder Keramik, um einen Antennenhüllkörper zu bilden, der in der Ausnehmung des Trägerelements aufgenommen ist. Damit ist in vorteilhafter Weise die Gefahr reduziert, dass kleine zu reinigende oder pflegende Instrumente durch die Ausnehmung fallen und verloren gehen.

Erfindungsgemäß ist die zumindest eine Koppelantenne als Flächenantenne mit einer Öffnung oder einem Durchbruch in einer Außenwand des Trägerelements oder als schlitzförmige Antenne oder Schlitzantenne ausgebildet. Eine Flächenantenne ermöglicht in vorteilhafter Weise eine sehr einfache Herstellung der Koppelantenne, zum Beispiel durch Formen einer Öffnung, eines Durchbruchs oder eines Schlitzes in einem Teil des Trägerelements.

Vorzugsweise weist die Flächenantenne einen länglichen, zum Beispiel geraden oder gebogenen oder rechteckigen oder spiralförmigen, Schlitz oder Durchbruch auf. Die unterschiedlichen Formen ermöglichen in vorteilhafter Weise die Richtung und/ oder Form und/ oder Intensität der abgestrahlten elektromagnetischen Strahlung zu variieren. Vorzugsweise ist der Schlitz oder Durchbruch in einer Außenwand des Trägerelements vorgesehen, zum Beispiel dem Boden oder der Bodenplatte, einer Seitenwand oder der Abdeckung.

Bevorzugt ist der Schlitz oder Durchbruch mit einem elektrisch nichtleitenden Material, zum Beispiel Kunststoff oder Glas oder Keramik, gefüllt, um insbesondere einen durchgängigen oder kontinuierlichen Trägerelementkörper zu bilden. Damit ist in vorteilhafter Weise die Gefahr reduziert, dass kleine zu reinigende oder pflegende Instrumente durch den Schlitz fallen und verloren gehen.

Vorzugsweise ist die Länge der Koppelantenne, insbesondere des Schlitzes oder Durchbruchs, auf die Wellenlänge oder Frequenz der Daten und/ oder Energie aufweisenden, zu übertragenden elektromagnetischen Strahlung abgestimmt, insbesondere auf eine elektromagnetische Strahlung im UHF- und/ oder SHF-Frequenz- oder Wellenlängenbereich, vorzugsweise auf elektromagnetische Strahlung mit einer Wellenlänge von etwa 25 cm - 40 cm, bevorzugt 29 cm - 35 cm, besonders bevorzugt auf eine Wellenlänge von etwa 34,6 cm oder etwa 33 cm. Vorzugsweise weist der Schlitz oder Durchbruch eine halbe oder Viertel Länge der Länge zumindest einer zu übertragenden Wellenlänge auf, insbesondere der im Vorstehenden genannten Wellenlängen(bereiche), wodurch in vorteilhafter Weise eine zuverlässige Übertragung der Daten und/ oder Energie aufweisenden elektromagnetischen Strahlung gesichert ist. Zum Beispiel beträgt eine Länge des Schlitzes oder Durchbruchs zumindest etwa 6,25 cm, vorzugsweise zumindest etwa 7,5 cm, bevorzugt etwa 8,25 cm oder etwa 8,65 cm oder etwa 16,5 cm oder etwa 17,3 cm. Vorzugsweise weist der Schlitz eine längliche Form mit zumindest einer Langseite und einer Schmalseite auf.

Vorzugsweise ist die Öffnung oder der Durchbruch oder Schlitz der Flächenantenne größer, insbesondere erheblich größer als die Gitteröffnungen oder (kreisrunden) Öffnungen zum Befestigen des zumindest einen Positionierelements oder der zumindest einen Halterung oder die insbesondere als Durchlass für ein Reinigungs- oder Pflegemittel dienenden Öffnungen oder länglichen Öffnungen des Trägerelements. Besonders bevorzugt wirken diese Gitteröffnungen oder (länglichen) Öffnungen nicht als Koppelantenne für die zwischen der ersten und zweiten Sende- und Empfangseinheit übertragenen, Daten und/ oder Energie aufweisenden elektromagnetischen Strahlung, insbesondere für die Strahlung mit SHF- und/ oder UHF-Frequenz oder Wellenlänge. Besonders bevorzugt sind die Abmessungen dieser Gitteröffnungen oder (länglichen) Öffnungen so bemessen, dass sie nicht als Koppelantennen für die Wellenlängen oder Frequenzen der zwischen der ersten und zweiten Sende- und Empfangseinheit übertragenen, Daten und/ oder Energie aufweisenden elektromagnetischen Strahlung wirken, wobei die Wellenlänge der elektromagnetischen Strahlung insbesondere in den im Vorstehenden beschriebenen Bereichen liegt. Damit wird in vorteilhafter Weise ein unerwünschter Einfluss der Gitteröffnungen oder (länglichen) Öffnungen auf die Übertragung der elektromagnetischen Strahlung zwischen der ersten und zweiten Sende- und Empfangseinheit zumindest verringert oder vermieden.

Insbesondere ist die Anzahl der Öffnungen oder Durchbrüche oder Schlitze der Flächenantenne erheblich geringer als die Anzahl der Gitteröffnungen oder (kreisrunden) Öffnungen zum Befestigen des zumindest einen Positionierelements oder die insbesondere als Durchlass für ein Reinigungs- oder Pflegemittel dienenden (länglichen) Öffnungen des Trägerelements. Bevorzugt ist die Anzahl der Gitteröffnungen oder (kreisrunden oder länglichen) Öffnungen zumindest fünfmal, vorzugsweise zumindest zehnmal größer als die Anzahl der Flächenantennen oder der Öffnungen oder der Durchbrüche oder Schlitze der Flächenantenne. Vorzugsweise sind die Flächenantennen oder die Öffnungen oder Durchbrüche oder Schlitze der Flächenantenne von mehreren Gitteröffnungen oder (kreisrunden oder länglichen) Öffnungen des Trägerelements umgeben.

Vorzugsweise sind an einem Trägerelement mehrere Koppelantennen vorgesehen. Dadurch wird in vorteilhafter Weise die Übertragung der Daten und/ oder Energie aufweisenden elektromagnetischen Strahlung noch weiter verbessert, so wie dies gemäß verschiedener möglicher Varianten im Folgenden im Detail beschrieben ist.

Vorzugsweise sind die mehreren Koppelantennen an unterschiedlichen Abschnitten oder Wänden vorgesehen, zum Beispiel am Boden oder an der Grund- oder Gitterplatte und/ oder an einer der Seitenwände und/ oder an der Abdeckung. Damit wird die Übertragung der Daten und/ oder Energie aufweisenden elektromagnetischen Strahlung verbessert, wenn zum Beispiel mehrere zu reinigende oder pflegende medizinische oder dentale Instrument an dem Trägerelement angeordnet sind, deren erste Sende- und Empfangseinheiten in unterschiedliche Richtungen weisen.

Alternativ oder zusätzlich sind zwei der mehreren Koppelantennen ineinander angeordnet. Zum Beispiel weist zumindest eine der mehreren Koppelantennen eine Flächenantenne mit einem länglichen Schlitz in einer Außenwand des metallischen Trägerelements und eine andere der mehreren Koppelantennen eine (geometrisch) lineare Antenne auf, wobei die lineare Antenne in dem länglichen Schlitz der Flächenantenne angeordnet ist. Besonders bevorzugt ist die Öffnung oder der Schlitz der Flächenantenne mit einem Füllmaterial, insbesondere einem elektrisch nichtleitenden Füllmaterial, gefüllt, in das die lineare Antenne aufgenommen oder eingebettet ist. Diese Anordnung bewirkt in vorteilhafter Weise zusätzlich zur verbesserten Übertragung der elektromagnetischen Strahlung eine kompakte, platzsparende Anordnung der mehreren Koppelantennen.

Alternativ oder zusätzlich sind zumindest zwei dieser mehreren Koppelantennen zur Übertragung elektromagnetischer Strahlung unterschiedlicher Frequenzen ausgebildet. Insbesondere weisen diese Koppelantennen zur Übertragung unterschiedlicher Frequenzen unterschiedliche Abmessungen auf, insbesondere unterschiedliche Längen. Damit ist in vorteilhafter Weise ein Trägerelement geschaffen, das in mehreren Ländern, in denen unterschiedliche Frequenzen zur Daten und/ oder Energieübertragung freigegeben sind, verkauft und verwendet werden kann. Der Hersteller eines derartigen Trägerelements erspart sich somit die Herstellung verschiedener Trägerelemente für unterschiedliche Länder.

Vorzugsweise ist ein, insbesondere medizinisches oder dentales, Reinigungs- oder Pflegegerät für zumindest ein medizinisches oder dentales Instrument vorgesehen, das ein im Vorstehenden beschriebenes Trägerelement und eine zweite Sende- und Empfangseinheit aufweist.

Das Reinigungs- oder Pflegegerät umfasst vorzugsweise ein Gehäuse, wobei die zweite Sende- und Empfangseinheit in dem Gehäuse oder außerhalb des Gehäuses angeordnet ist.

Das, insbesondere medizinische oder dentale, Reinigungs- oder Pflegegerät umfasst vorzugsweise des Weiteren zumindest eines der folgenden Elemente: eine Fördervorrichtung zur Förderung eines Reinigungs- oder Pflegemediums; eine, insbesondere mit der Fördervorrichtung verbundene, Abgabevorrichtung zur Abgabe eines Reinigungs-oder Pflegemediums auf und / oder in das medizinische oder dentale Instrument; zumindest eine Kammer zur Aufnahme des zu reinigenden oder zu pflegenden medizinischen oder dentalen Instruments; eine Stellvorrichtung zum Stellen und/ oder Auswählen eines Betriebsparameters oder eines Betriebsprogramms; eine Steuerung oder einen Mikrocontroller zur Steuerung des Reinigungs- oder Pflegegeräts, insbesondere des Betriebs des Reinigungs- oder Pflegegeräts; einen Verdampfer zum Erzeugen von als Reinigungsmedium dienenden Dampf.

Vorzugsweise umfasst die Fördervorrichtung des Reinigungs- oder Pflegegeräts zumindest eines der folgenden Bauteile: eine Pumpe; einen Anschluss an eine Medienquelle, insbesondere an einen Behälter; einen Anschluss an eine Druckluftleitung; ein Ventil; eine Förderleitung zur Förderung des Reinigungs- oder Pflegemediums, insbesondere von einer Medienquelle, zum Beispiel einem Behälter zur Lagerung eines Reinigungs- oder Pflegemediums, zu einem zu reinigenden oder zu pflegenden Instrument.

Vorzugsweise ist die Abgabevorrichtung zur Abgabe eines Reinigungs- oder Pflegemediums ausgebildet, das Reinigungs- oder Pflegemedium an die Außenseite und / oder an die Innenseite des zu reinigenden oder zu pflegenden Instruments abzugeben.

Vorzugsweise umfasst das, insbesondere medizinische oder dentale, Reinigungs-oder Pflegegerät eine der folgenden Vorrichtungen: einen Sterilisator; eine thermische oder chemische Desinfektionsvorrichtung; eine Reinigungs- und/ oder Desinfektionsvorrichtung (in ihrem Aufbau ähnlich einer Geschirrspülmaschine); eine Pflegevorrichtung zur Abgabe eines Pflegemittels, zum Beispiel eines Schmiermittels. Das Reinigungs- oder Pflegegerät ist somit ausgebildet, das zumindest eine zu reinigenden oder zu pflegenden Instrument innen und/ oder außen zu reinigen, zu desinfizieren, zu sterilisieren und/ oder mit einem Schmiermittel zu versorgen.

Gemäß einem Ausführungsbeispiel ist eine Kombination vorgesehen, bestehend aus zumindest einem, insbesondere im Vorstehenden beschriebenen medizinischen oder dentalen Instrument mit einer ersten Sende- und Empfangseinheit und einem im Vorstehenden beschriebenen Trägerelement, wobei die zumindest eine Koppelantenne des Trägerelements ausgebildet ist, eine drahtlos zwischen der ersten Sende- und Empfangseinheit und einer zweiten Sende- und Empfangseinheit übertragbare, Daten und/ oder Energie aufweisende elektromagnetische Strahlung zu empfangen und wiederum abzustrahlen, um die drahtlose Übertragung der elektromagnetischen Strahlung zwischen der ersten Sende- und Empfangseinheit und der zweiten Sende- und Empfangseinheit zu unterstützen. Vorzugsweise umfasst die Kombination auch ein insbesondere im Vorstehenden beschriebenes Reinigungs- oder Pflegegerät.

Erfindungsgemäß ist vorgesehen: die Verwendung eines im Vorstehenden beschriebenen Trägerelements bei der Reinigung oder Pflege zumindest eines medizinischen oder dentalen Instruments in einem Reinigungs- oder Pflegegerät zur Unterstützung einer drahtlosen Übertragung von Daten und/ oder Energie aufweisender elektromagnetischer Strahlung zwischen einer ersten, an dem medizinischen oder dentalen Instrument angeordneten Sende- und Empfangseinheit und einer zweiten Sende- und Empfangseinheit. Die Verwendung des Trägerelements erfolgt insbesondere in oder mit einem im Vorstehenden beschriebenen Reinigungs- oder Pflegegerät und/ oder mit einem im Vorstehenden beschriebenen medizinischen oder dentalen Instrument mit einer ersten Sende- und Empfangseinheit.

Erfindungsgemäß ist ein Verfahren zur drahtlosen Übertragung von Daten und/ oder Energie aufweisender elektromagnetischer Strahlung zwischen einer ersten, an einem medizinischen oder dentalen Instrument angeordneten Sende- und Empfangseinheit und einer zweiten Sende- und Empfangseinheit, vorgesehen. Dabei wird ein Trägerelement zum Lagern des medizinischen oder dentalen Instruments zur Verfügung gestellt wird, wobei an dem Trägerelement zumindest eine Koppelantenne vorgesehen ist, die ausgebildet ist, die drahtlos zwischen der ersten Sende- und Empfangseinheit und der zweiten Sende- und Empfangseinheit übertragene elektromagnetische Strahlung zu empfangen und wiederum abzustrahlen, um die drahtlose Übertragung der Daten und/ oder Energie aufweisenden elektromagnetischen Strahlung zwischen der ersten Sende- und Empfangseinheit und der zweiten Sende- und Empfangseinheit zu unterstützen. Die zumindest eine Koppelantenne weist eine Flächenantenne auf, die durch eine Öffnung oder einen Durchbruch oder einen Schlitz in einer Außenwand des Trägerelements oder in einer in das Trägerelement eingelegten Platte gebildet ist. Das Trägerelement mit dem darauf gelagerten medizinischen oder dentalen Instrument mit der ersten Sende- und Empfangseinheit wird in ein, insbesondere medizinisches oder dentales, Reinigungs- oder Pflegegerät zum Reinigen oder Pflegen des medizinischen oder dentalen Instruments eingebracht. Die Daten und/ oder Energie aufweisende elektromagnetische Strahlung wird drahtlos mittels der zumindest einen Koppelantenne zwischen der ersten Sende- und Empfangseinheit und der zweiten Sende- und Empfangseinheit übertragen. Bevorzugt umfasst das Trägerelement ein im Vorstehenden beschriebenes Trägerelement. Bevorzugt wird das Verfahren in oder mit einem im Vorstehenden beschriebenen Reinigungs- oder Pflegegerät durchgeführt.

Vorzugsweise wird die Daten und/ oder Energie aufweisende elektromagnetische Strahlung von der zumindest einen Koppelantenne zu der ersten Sende- und Empfangseinheit übertragen und/ oder es wird die elektromagnetische Strahlung von der ersten Sende- und Empfangseinheit zu der zumindest einen Koppelantenne übertragen.

Vorzugsweise ist vorgesehen, dass die Übertragung der Daten und/ oder Energie aufweisenden elektromagnetischen Strahlung vor, während oder nach dem Einbringen des Trägerelements mit dem darauf gelagerten, zu reinigenden oder zu pflegenden medizinischen oder dentalen Instrument mit der ersten Sende- und Empfangseinheit in das Reinigungs- oder Pflegegerät, insbesondere in die Kammer zur Aufnahme des zu reinigenden oder zu pflegenden medizinischen oder dentalen Instruments, stattfindet. Vorzugsweise ist vorgesehen, dass die Daten und/ oder Energie aufweisende elektromagnetische Strahlung vor, während oder nach dem Schließen oder Verriegeln einer Öffnung des Reinigungs- oder Pflegegeräts, durch welche das Trägerelement mit dem darauf gelagerten, zu reinigenden oder zu pflegenden medizinischen oder dentalen Instrument mit der ersten Sende- und Empfangseinheit in das Reinigungs- oder Pflegegerät eingebracht wird, übertragen wird. Die Öffnung ist insbesondere mit der Kammer zur Aufnahme des zu reinigenden oder zu pflegenden medizinischen oder dentalen Instruments verbunden. Die Öffnung ist insbesondere durch einen Deckel oder eine Tür verschließbar oder verriegelbar. Der Deckel oder die Tür ist insbesondere beweglich, vorzugsweise drehbar oder verschwenkbar, an dem Reinigungs- oder Pflegegerät, zum Beispiel an dem Gehäuse, befestigt. Besonders bevorzugt wird die Übertragung der Daten und/ oder Energie aufweisenden elektromagnetischen Strahlung nach Abschluss der Reinigung oder Pflege des medizinischen oder dentalen Instruments durchgeführt. Besonders bevorzugt ist die Übertragung der Daten und/ oder Energie aufweisenden elektromagnetischen Strahlung vor Öffnen des Deckels oder der Tür zur Entnahme des gereinigten oder gepflegten Instruments abgeschlossen. Durch diese Maßnahmen ist in vorteilhafter Weise sichergestellt, dass nur dann elektromagnetische Strahlung, insbesondere Daten, besonders bevorzugt ein einem Instrument eindeutig zuordenbarer Identifizierungscode, übertragen werden, wenn das zu reinigende oder pflegende Instrument in das Reinigungs- oder Pflegegerät eingebracht wurde bzw. wenn die Reinigung oder Pflege durchgeführt wurde.

Vorzugsweise ist vorgesehen, dass das zumindest eine medizinische oder dentale Instrument mittels eines (mechanischen) Positionierelements, insbesondere einer Halterung, an dem Trägerelement fixiert wird, insbesondere mittels eines im Vorstehenden beschriebenen Positionierelements. Besonders bevorzugt wird das medizinische oder dentale Instrument an der Halterung oder durch das Positionierelement derart in einer bestimmten Position fixiert, dass die erste Sende- und Empfangseinheit zu der zumindest einen Koppelantenne gewandt ist und/ oder sich in Richtung der zumindest einen Koppelantenne erstreckt, wodurch in vorteilhafter Weise die drahtlose Kommunikation, insbesondere die Übertragung der elektromagnetischen Strahlung, zwischen der ersten Sende- und Empfangseinheit und der zumindest einen Koppelantenne verbessert wird.

Die Erfindung wird nachfolgend anhand bevorzugter Ausführungsbeispiele und Bezug nehmend auf die beigefügten Zeichnungen erläutert.
Figur 1 zeigt ein Trägerelement zum Lagern zumindest eines medizinischen oder dentalen Instruments in einem Reinigungs- oder Pflegegerät in Form eines Trays mit einer in einem Boden des Trays angeordneten, schlitzförmigen Koppelantenne.
Figur 2 zeigt ein Trägerelement in Form eines Trays mit mehreren in einem Boden des Trays angeordneten, schlitzförmigen Koppelantennen.
Figur 3 zeigt ein Trägerelement in Form eines Trays mit einer in einem Boden des Trays angeordneten, schlitzförmigen, gebogenen Koppelantenne.
Figur 4 zeigt ein Trägerelement in Form eines Trays mit mehreren in einem Boden des Trays gewinkelt zueinander angeordneten, schlitzförmigen Koppelantennen.
Figur 5 zeigt ein Trägerelement zum Lagern zumindest eines medizinischen oder dentalen Instruments in einem Reinigungs- oder Pflegegerät in Form einer Kassette mit mehreren in einem Boden und in einer Abdeckung der Kassette vorgesehenen Koppelantennen.
Figur 6 zeigt ein Trägerelement in Form einer Kassette mit mehreren in einem Boden und in einer Abdeckung der Kassette vorgesehenen, gebogenen Koppelantennen.
Figur 7 zeigt ein Trägerelement in Form einer Kassette mit einer in einer Seitenwand der Kassette vorgesehenen Koppelantenne.
Figur 8 zeigt ein Trägerelement in Form einer Kassette mit einer in einer Seitenwand der Kassette vorgesehenen schlitzförmigen Koppelantenne, in der eine linear Koppelantenne aufgenommen ist.
Figur 9 zeigt ein Trägerelement in Form eines Trays mit einer in einem Boden des Trays angeordneten, schlitzförmigen Koppelantenne, in der eine linear Koppelantenne aufgenommen ist.
Figur 10 zeigt ein Trägerelement in Form eines Trays mit einer in das Tray eingesetzten Bodenplatte aus elektrisch nichtleitendem Material.
Figur 11 zeigt ein Trägerelement in Form eines Trays mit einer in das Tray eingesetzten Bodenplatte aus elektrisch nichtleitendem Material, in der eine lineare Koppelantenne aufgenommen ist.
Figur 12 zeigt ein Ausführungsbeispiel eines Reinigungs- oder Pflegegeräts für zumindest ein medizinisches oder dentales Instrument zur Verwendung mit einem Trägerelement mit zumindest eine Koppelantenne.

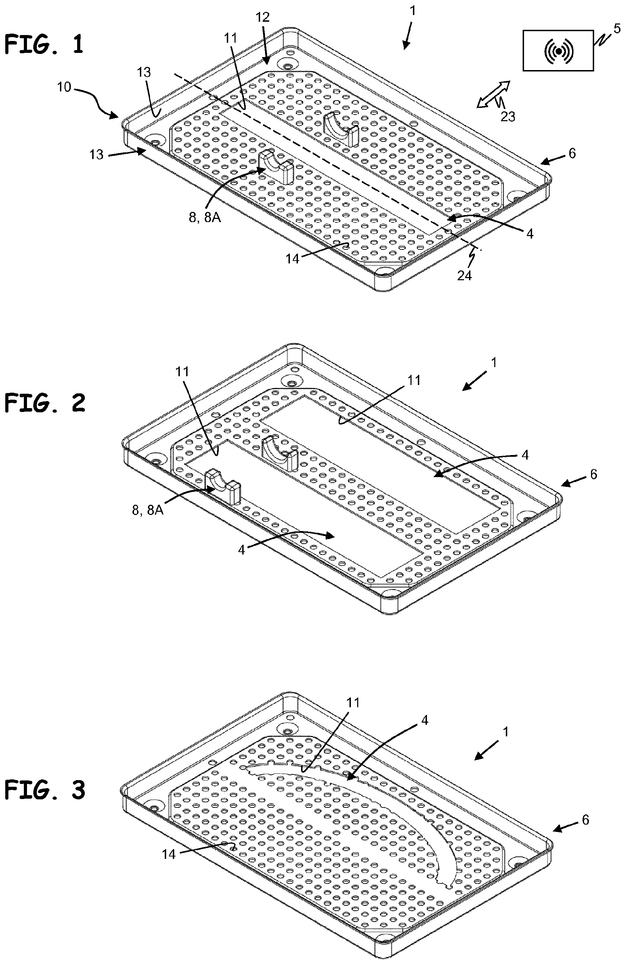

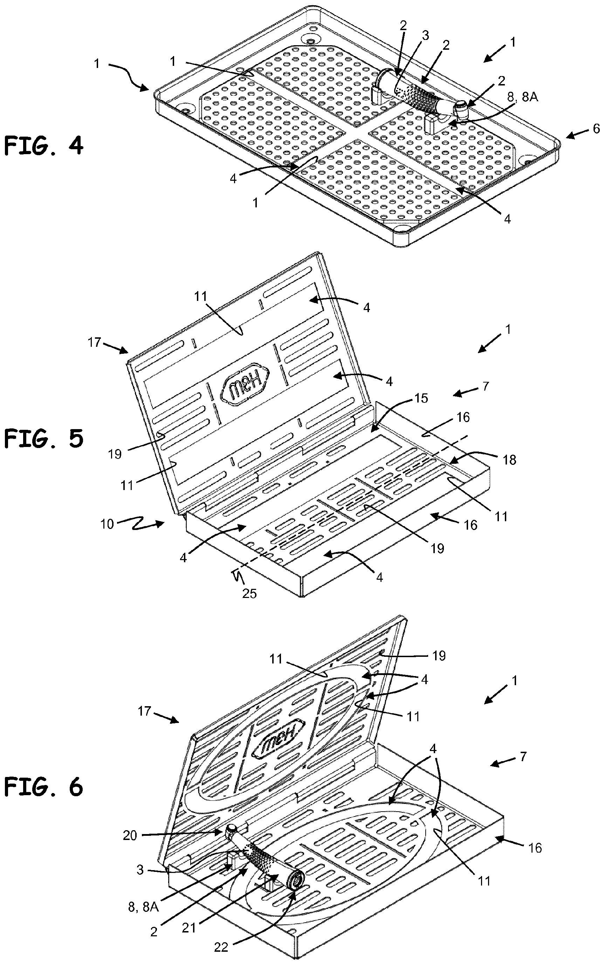

Die Figuren 1 ― 11 zeigen unterschiedliche Trägerelemente 1 zum Lagern zumindest eines medizinischen oder dentalen Instruments 2 (siehe Figuren 4 und 6) in einem Reinigungs- oder Pflegegerät 50, um das zumindest eine medizinische oder dentale Instrument 2 in dem Reinigungs- oder Pflegegerät 50 zu reinigen oder zu pflegen. Das Trägerelemente 1 ist zur Aufnahme in dem Reinigungs- oder Pflegegerät 50 vorgesehen.

Die Figuren 1 ― 4 und 9 ― 11 zeigen ein als Tray 6 ausgebildetes Trägerelement 1. Der Tray 6 umfasst mehrere Außenwände 10, zum Beispiel einen flachen und/ oder plattenförmigen Boden 12 und mehrere damit verbundene und die Bodenplatte 12 umgebende Seitenwände 13. Der Boden 12 umfasst eine Vielzahl von Öffnungen 14 oder Sieböffnungen, die als Durchlass für ein Reinigungs- oder Pflegemittel dienen. Die Öffnungen 14 sind vorzugsweise rund, oval oder als Polygon, dessen Ecken alle auf einem gemeinsamen Umfang angeordnet sind, ausgebildet.

Die Figuren 5 ― 8 zeigen ein als Kassette 7 ausgebildetes Trägerelement 1. Die Kassette 7 umfasst mehrere Außenwände 10, zum Beispiel einen Boden 15, mehrere damit verbundene und den Boden 15 umgebende Seitenwände 16 und einen Deckel 17, der relativ zu dem Boden 15 und den Seitenwänden 16 verschwenkbar ausgebildet ist. Der Boden 15, die Seitenwände 16 und der Deckel 17 grenzen einen Innenraum 18 der Kassette 7 gegenüber der Umgebung ab. An dem Boden 15 und an dem Deckel 17 sind eine Vielzahl von Öffnungen 19, vorzugsweise längliche Öffnungen, vorgesehen, die als Durchlass für ein Reinigungs- oder Pflegemittel dienen.

An dem Trägerelement 1, 6, 7 kann zumindest ein Positionierelement 8, insbesondere eine Halterung 8A, vorgesehen sein, um das zumindest eine medizinische oder dentale Instrument 2 an dem Trägerelement 1, 6, 7 zu positionieren und/ oder zu befestigen (siehe insbesondere Figuren 1, 2, 4, 6 und 9). Alternativ ist es auch möglich an dem Trägerelement 1, 6, 7 kein Positionierelement 8 vorzusehen oder dieses aus dem Trägerelement 1, 6, 7 zu entfernen (wenn das Positionierelement 8 und das Trägerelement 1, 6, 7 voneinander lösbar ausgebildet sind), so dass das zumindest eine medizinische oder dentale Instrument 2 lose oder ohne definierte Position oder Befestigung in dem Trägerelement 1, 6, 7 aufgenommen ist.

Zur lösbaren Verbindung des zumindest einen Positionierelements 8, 8A mit dem Trägerelement 1, 6, 7 ist insbesondere vorgesehen, ein Verbindungselement des Positionierelements 8, 8A, zum Beispiel einen Fortsatz, in einer der Öffnungen 14, 19 des Trägerelement 1, 6, 7 einzufügen.

Das zumindest eine Positionierelement 8 ist als Halterung 8A mit einer Steckaufnahme oder einem Klemmelement ausgebildet, in welche das zu reinigende oder pflegende medizinische oder dentale Instrument 2 einsteckbar ist. Das Positionierelement 8 ist insbesondere derart ausgebildet, dass das medizinische oder dentale Instrument 2 in einer bestimmten oder vordefinierten Position, Orientierung und/ oder Anordnung in Bezug auf das Trägerelement 1, 6, 7 und/ oder eine Koppelantenne 4 des Trägerelements 1, 6, 7 angeordnet ist, so dass vorzugsweise eine Sende- und Empfangseinheit 3 des Instruments 2 der zumindest einen Koppelantenne 4 zugewandt ist.

In den Figuren 4 und 6 ist ein zu reinigendes oder pflegendes Instrument 2 dargestellt, das durch ein Positionierelement 8, 8A an dem Trägerelement 1 positioniert und befestigt ist. Das Instrument 2 ist als dentales Hand- oder Winkelstück ausgebildet. Das Hand- oder Winkelstück 2 umfasst einen Kopfabschnitt 20, der eine Werkzeughalterung zum Befestigen eines Behandlungswerkzeugs und/ oder eine Behandlungsmedien-Abgabevorrichtung aufweist, und einen an den Kopfabschnitt 20 angrenzenden, vorzugsweise entlang einer Längsachse gebogenen, Griffabschnitt 21. Der Griffabschnitt 21 endet vorzugsweise in einem Kupplungsanschluss 22 zur Verbindung des Instrument 2 mit einer Steuer- und/ oder Versorgungseinheit zur Versorgung mit Energie und/ oder einem Behandlungsmedium.

An dem Instrument 2 ist eine erste Sende- und Empfangseinheit 3, zum Beispiel ein RFID-Etikett, zur drahtlosen Übertragung von Daten und/ oder Energie aufweisender elektromagnetischer Strahlung, insbesondere im UHF- und/ oder SHF-Frequenz- oder Wellenlängenbereich, angeordnet. Die erste Sende- und Empfangseinheit 3 kann außen an dem Instrument 2, insbesondere an dessen Außenhülse, oder im Innen des Instruments 2, insbesondere in dessen hohlen Außenhülse, angeordnet sein. Die erste Sende- und Empfangseinheit 3 kann im Kopfabschnitt 20 oder im Griffabschnitt 21, sowohl in jenem Teil des Griffabschnitts 21, der sich zwischen dem Kopfabschnitt 20 und der Biegung erstreckt, oder in jenem Teil des Griffabschnitts 21, der sich zwischen der Biegung und dem Kupplungsanschluss 22 erstreckt, oder direkt im Bereich der Biegung des Griffabschnitts 21 angeordnet sein.

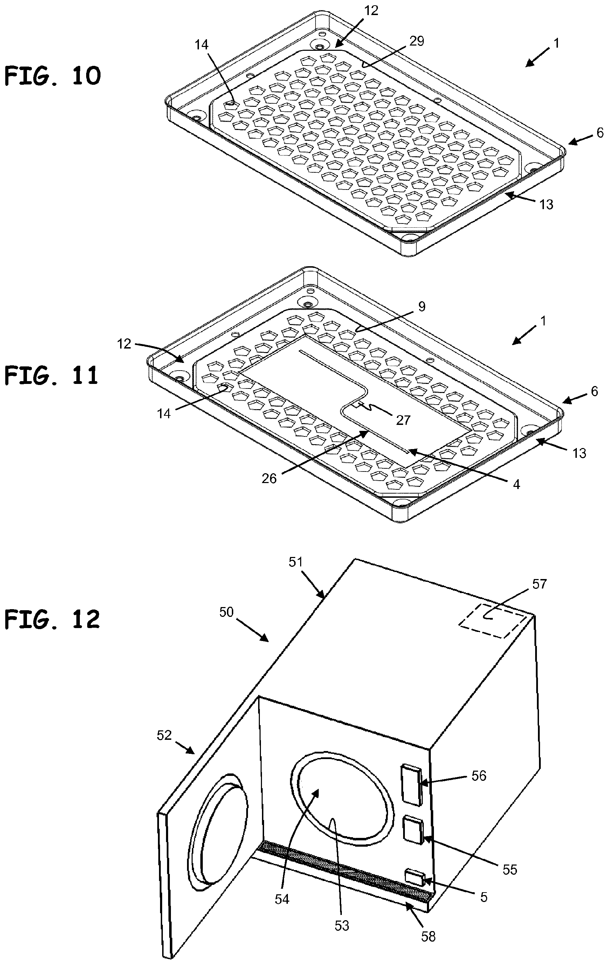

Die erste Sende- und Empfangseinheit 3 und eine davon beabstandete zweite Sende- und Empfangseinheit 5 (siehe Figur 1) sind ausgebildet, kommunikativ in Verbindung 23 zu treten, um eine drahtlose Übertragung von Daten und/ oder Energie aufweisender elektromagnetischer Strahlung, insbesondere im UHF- und/ oder SHF-Frequenz- oder Wellenlängenbereich, zwischen den Sende- und Empfangseinheiten 3, 5 zu bewirken. Die zweite Sende- und Empfangseinheit 5 ist zum Beispiel als Lese- und/ oder Empfangsgerät ausgebildet. Die zweite Sende- und Empfangseinheit 5 kann entweder als separates, eigenständiges Gerät (siehe Figur 1) oder als (integraler) Teil eines mit dem medizinischen oder dentalen Instrument 2 operativ und/ oder kommunikativ in Verbindung stehenden Gerät, zum Beispiel dem Reinigungs- oder Pflegegerät 50 (siehe Figur 12), ausgebildet sein.

Zwischen der ersten und zweiten Sende- und Empfangseinheit 3, 5 übertragene Energie umfasst insbesondere Energie zum Betrieb der ersten Sende- und Empfangseinheit 3. Zwischen der ersten und zweiten Sende- und Empfangseinheit 3, 5 übertragene Daten umfassen insbesondere Daten zur Identifizierung des Instruments 2, zum Beispiel einen Code zur Identifizierung des Instruments 2, und optional weitere Daten, zum Beispiel Daten betreffend den Reinigungs- oder Pflegezustand des Instruments 2, die Anzahl und/ oder Art der Reinigungs- oder Pflegeprozesse, die das Instrument 2 bereits durchlaufen hat oder noch durchlaufen darf, ein Betriebsprogramm oder zumindest einen Betriebsparameter, das/ die bei der Reinigungs- oder Pflege des Instruments 2 an dem Reinigungs- oder Pflegegerät 50 automatisch eingestellt werden sollen, und ähnliche Daten.

Die Trägerelemente 1, 6, 7 der Figuren 1 - 9 und 11 weisen zumindest eine Koppelantenne 4 auf, die ausgebildet ist, die drahtlos zwischen der ersten und zweiten Sende- und Empfangseinheit 3, 5 übertragene elektromagnetische Strahlung zu empfangen und wiederum abzustrahlen, um die drahtlose Übertragung der Daten und/ oder Energie aufweisenden elektromagnetischen Strahlung zwischen der ersten und zweiten Sende- und Empfangseinheit 3, 5 zu unterstützen oder zu verbessern. Die zumindest eine Koppelantenne 4 beeinflusst zum Beispiel die Abstrahlrichtung der Daten und/ oder Energie aufweisenden elektromagnetischen Strahlung und/ oder erhöht die Energiedichte der elektromagnetischen Strahlung und/ oder bewirkt eine Bündelung der elektromagnetischen Strahlung.

Die zumindest eine Koppelantenne 4 kann eine Vielzahl von unterschiedlichen Abmessungen und/ oder Formen und/ oder Ausführungen umfassen, von denen einige beispielhaft in den Figuren 1 - 9 und 11 dargestellt und im Folgenden beschrieben sind. Allen erfindungsgemäßen Koppelantennen 4, die ausgebildet sind, die drahtlos zwischen der ersten und zweiten Sende- und Empfangseinheit 3, 5 übertragene elektromagnetische Strahlung zu empfangen und wiederum abzustrahlen, um die drahtlose Übertragung der Daten und/ oder Energie aufweisenden elektromagnetischen Strahlung zwischen der ersten und zweiten Sende- und Empfangseinheit 3, 5 zu unterstützen, ist jedoch gemein, dass sie derart ausgebildet, insbesondere bemessen und geformt sind, dass sie auf die Wellenlänge oder Frequenz der zwischen der ersten und zweiten Sende- und Empfangseinheit 3, 5 zu übertragenden oder übertragenen elektromagnetischen Strahlung abgestimmt sind, insbesondere auf eine elektromagnetische Strahlung im UHF- und/ oder SHF-Frequenz-oder Wellenlängenbereich. Vorzugsweise sind die erfindungsgemäßen Koppelantennen 4 derart ausgebildet, dass die Übertragung der elektromagnetischen Strahlung zwischen der ersten und zweiten Sende- und Empfangseinheit 3, 5 aufgrund der Wirkung der Koppelantennen 4 im Vergleich zu einer Übertragung der elektromagnetischen Strahlung ohne den Koppelantennen 4 verbessert ist. Zum Beispiel ist aufgrund der Wirkung der Koppelantennen 4 während der drahtlosen Übertragung (und im Vergleich zur Übertragung der elektromagnetischen Strahlung ohne Koppelantenne 4) zumindest einer der folgenden Effekte erzielbar: ein verringerter Einfluss von Störgrößen auf die elektromagnetische Strahlung bzw. deren Übertragung; ein geringerer Verlust an durch die elektromagnetische Strahlung übertragenen Daten und/ oder übertragener Energie; ein geringeres Rauschen bei den übertragenen Daten; eine (pro Zeiteinheit) höhere Menge an übertragener Energie und/ oder Daten; eine (pro Zeiteinheit) raschere Übertragung einer bestimmten Menge an Energie und/ oder Daten.

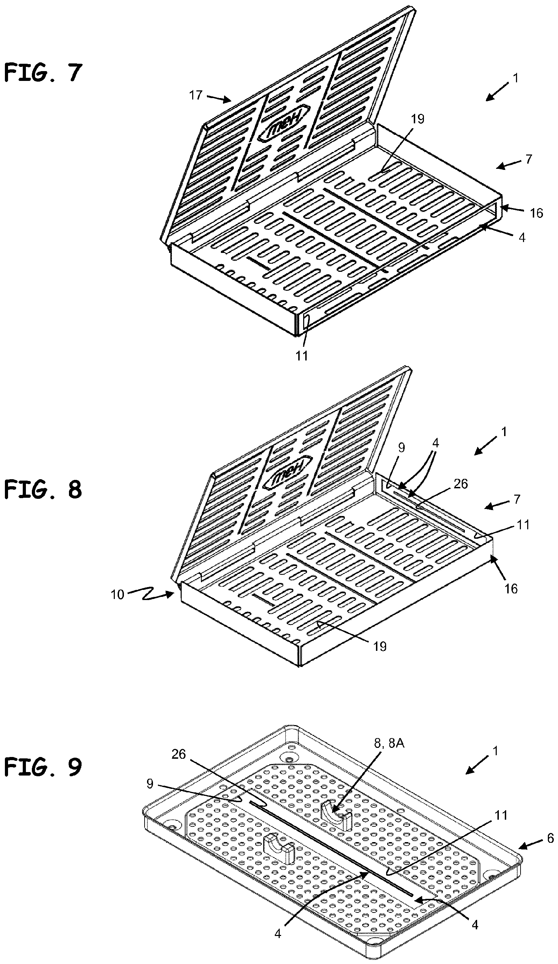

Die in den Figuren 1 - 9 und 11 gezeigten Koppelantennen 4 umfassen entweder nur zumindest eine Flächenantenne (siehe Figuren 1 - 7) oder nur zumindest eine (geometrisch) lineare Antenne, deren elektrischer Leiter 26 insbesondere einen metallischen Draht oder einen metallischen Stab aufweist (siehe Figur 11), oder zumindest eine Flächenantenne und eine lineare Antenne, deren elektrischer Leiter 26 insbesondere einen metallischen Draht oder einen metallischen Stab aufweist (siehe Figuren 8, 9).

Die in den Figuren 1 - 4, 9 gezeigten Trays 6 umfassen eine oder mehrere als Flächenantennen ausgebildete Koppelantennen 4. Die Flächenantennen sind als längliche Schlitze 11 in einer Außenwand 10, d.h. im Boden 12, des Trägerelements 1, 6 geformt. Zumindest der Boden 12 des Trays 6, vorzugsweise der gesamte Tray 6, ist aus Metall gefertigt, wodurch die drahtlose Übertragung der Daten und/ oder Energie aufweisenden elektromagnetischen Strahlung zusätzlich verbessert wird.

Die in den Figuren 5 - 8 gezeigten Kassetten 7 umfassen eine oder mehrere als Flächenantennen ausgebildete Koppelantennen 4. Die Flächenantennen sind als längliche Schlitze 11 in einer Außenwand 10, d.h. im Boden 15 und/ oder im Deckel 17 und/ oder in einer Seitenwand 16 (Langseite oder Schmalseite), des Trägerelements 1, 7 geformt. Zumindest jene Außenwand 10 der Kassette 7, in welcher eine Flächenantenne vorgesehen ist, vorzugsweise die gesamte Kassette 7, ist aus Metall gefertigt, wodurch die drahtlose Übertragung der Daten und/ oder Energie aufweisenden elektromagnetischen Strahlung zusätzlich verbessert wird.

Vorzugsweise sind die Positionierelemente 8, 8A derart auf dem Tray 6 oder auf der Kassette 7 angeordnet, dass ein darin positioniertes medizinisches oder dentales Instrument 2 über eine Flächenantenne oder einen Schlitz 11 ragt und sich insbesondere die erste Sende- und Empfangseinheit 3 nahe oder über der Flächenantenne oder dem Schlitz 11 befindet (siehe insbesondere Figuren 4 und 6; eine derartige Anordnung der Positionierelemente 8, 8A ist entsprechend auch für die Figuren 1 - 3, 5, 9 möglich). Damit wird die drahtlose Übertragung der Daten und/ oder Energie aufweisenden elektromagnetischen Strahlung noch weiter verbessert.

Der Schlitz 11 ist als die Außenwand 10 (12; 15 - 17) durchsetzender Durchlass oder durchsetzende Öffnung gebildet, der/ die insbesondere keinen Festkörper enthält. Alternativ kann der Schlitz 11 mit einem elektrisch nichtleitendem Material, zum Beispiel Kunststoff oder Glas oder Keramik, gefüllt sein, um eine kontinuierliche Außenwand 10, insbesondere einen kontinuierlichen Boden 12, 15, zu bilden, um zu vermeiden, das zu reinigende oder pflegende Instrumente 2 durch den Schlitz 11 fallen und gegebenenfalls verloren gehen. Das elektrisch nichtleitende Material ist zum Beispiel als durchgängige oder mit Öffnungen versehene Platte ausgebildet, die den Schlitz 11 teilweise oder vollständig ausfüllt.

Die Schlitze 11 der Figuren 1, 2, 4, 5 und 7 - 9 sind gerade und/ oder im Wesentlichen rechteckig ausgebildet. Die Schlitze 11 der Figur 3 und 6 sind gebogen ausgebildet, wodurch der Schlitz 11 länger sein kann als die Langseite des Trays 6 oder der Kassette 7, um zum Beispiel die für die zu übertragende Wellenlänge der elektromagnetischen Strahlung passende Länge aufzuweisen. Ein gebogener Schlitz 11 kann neben der in den Figuren 3, 6 dargestellten Kreisbogenform eine Vielzahl anderer Formen aufweisen, zum Beispiel S-förmig, spiralförmig oder ähnliche Formen.

Der Schlitz 11 oder zumindest einer der Schlitze 11 der Flächenantennen kann in Bezug auf eine Längsachse 24 des Trays 6 oder in Bezug auf eine Längsachse 25 der Kassette 7 unterschiedlich angeordnet sein und eine oder mehrere der folgenden Anordnungen aufweisen: mittig im Trägerelement 1, 6, 7 so dass insbesondere die Längsachse 24 auch die Mittelachse des Schlitzes 11 bildet (siehe Figuren 1, 4 - langer Schlitz, 9); außermittig (siehe Figuren 2, 3, 4 - kurzer Schlitz, 5 - 8) und vorzugsweise parallel zur Längsachse 24 (siehe Figuren 2, 5 - 8), insbesondere wenn mehrere Schlitze 11 vorhanden sind; gewinkelt, zum Beispiel orthogonal, zur Längsachse 24 (siehe Figuren 3, 4 - kurzer Schlitz, 6). Durch die unterschiedliche Anordnung und Form der Schlitze 11 können Trägerelemente 1, 6, 7 geschaffen werden, die in vorteilhafter Weise an bestimmte Instrumente 2 angepasst sind, zum Beispiel an die Länge des Instruments 2 und/ oder an die Position der ersten Sende- und Empfangseinheit 3 am Instrument 2.

Die Trägerelemente 1, 6, 7 der Figuren 1, 3, 7, 11 weisen eine einzige Koppelantenne 4 auf, die entweder als Flächenantenne, insbesondere mit einem Schlitz 11 (siehe Figuren 1, 3, 7), oder als metallische, lineare Antenne mit einem stab- oder drahtförmigen elektrischen Leiter 26 (siehe Figur 11) ausgebildet ist.

Die Trägerelemente 1, 6, 7 der Figuren 2, 4 - 6, 8, 9 weisen mehrere Koppelantennen 4 auf, die entweder nur als schlitzförmigen Flächenantennen 11 ausgebildet sind (siehe Figuren 2, 4 - 6) oder zumindest eine schlitzförmige Flächenantenne 11 und zumindest eine metallische, lineare Antenne mit einem stab- oder drahtförmigen elektrischen Leiter 26 aufweist (siehe Figuren 8 - 9). Selbstverständlich ist es auch möglich ein Trägerelement 1, 6, 7 mit ausschließlich mehreren metallischen, linearen Antennen mit jeweils einem stab- oder drahtförmigen elektrischen Leiter 26 herzustellen. Wie aus den Figuren erkennbar können die mehreren Koppelantennen 4 entweder alle an einer einzigen und/ oder gemeinsamen Außenwand 10, insbesondere an dem Boden 12, 15 oder einer Seitenwand 16 (siehe Figuren 2, 4, 8, 9), oder an unterschiedlichen Außenwänden 10, insbesondere an dem Boden 12, 15 und dem Deckel 17 (siehe Figuren 5, 6), vorgesehen sein.

Bei den Figuren 8, 9 ist die metallische, lineare Antenne in einer Ausnehmung 9 angeordnet, wobei diese Ausnehmung 9 durch den Schlitz 11 der Flächenantenne gebildet ist, wodurch in vorteilhafter Weise eine einfachere Herstellung des Trägerelements 1 mit den beiden Koppelantennen 4 möglich ist. Der Schlitz 11 ist hierbei zumindest teilweise mit einem elektrisch nichtleitenden Material gefüllt (so wie im Vorstehenden bereits beschrieben), in dem die metallische, lineare Antenne aufgenommen oder eingebettet ist. Selbstverständlich ist es jedoch auch möglich, die durch den Schlitz 11 gebildete Flächenantenne und die metallische, lineare Antenne beabstandet voneinander anzuordnen, entweder an derselben Außenwand 10 oder an unterschiedlichen Außenwänden 10. Bei getrennter, voneinander beabstandeter Anordnung der beiden Koppelantennen 4 ist die metallische, lineare Antenne somit in einer eigenen, separaten Aufnahme 9 angeordnet.

Bei Vorhandensein mehrerer gleichartiger Koppelantennen 4, d.h. ausschließlich von Flächenantennen oder ausschließlich von metallischen, linearen Antennen, an einem Trägerelement 1 können die mehreren, gleichartigen Koppelantennen 4 ident ausgebildet sein (siehe Figuren 2, 5, 6) oder sie können sich in zumindest einem Parameter oder einer Eigenschaft unterscheiden, zum Beispiel in Bezug auf ihre Form und Abmessungen (siehe Figur 4). Die beiden Schlitzantennen 11 des Trays 6 der Figur 4 unterscheiden sich beispielsweise in ihrer Länge. Gleichartige Koppelantennen 4 mit zumindest einer unterschiedlichen Eigenschaft können selbstverständlich in entsprechender Weise auch an einer Kassette 7 vorgesehen sein.

Das Vorsehen mehrerer Koppelantennen 4 an einem Trägerelement 1 bietet mehrere Vorteile: so können zum Beispiel unterschiedliche Frequenzen oder Wellenlängen der elektromagnetischen Strahlung übertragen werden, wenn die Koppelantennen 4 unterschiedliche Längen aufweisen (siehe Figuren 4, 8, 9) und/ oder eine exakte Positionierung des zumindest einen Instruments 2 relativ zu den Koppelantennen 4 ist weniger kritisch, da aufgrund der mehreren Koppelantennen 4 sichergestellt ist, dass die erste Sende- und Empfangseinheit 3 des Instruments 2 zumindest ausreichend nahe zu einer der mehreren Koppelantennen 4 positioniert ist (siehe Figuren 4 - 6).

Das Vorsehen der zumindest einen Schlitzantenne 11 in einer Seitenwand 16 (siehe Figuren 7, 8) verringert im Vergleich zur Anordnung des Schlitzes 11 im Boden 12, 15 die Gefahr, dass das zumindest eine Instrument 2 ungewollt durch einen Schlitz 11 fällt. Die Anordnung einer Schlitzantenne 11 in einer Seitenwand 13 ist in entsprechender Weise für ein Tray 6 denkbar.

Die Figur 11 zeigt ein Tray 6 mit einer metallischen, linearen Antenne, die in einer Ausnehmung 9 angeordnet ist. Im Gegensatz zu den Ausführungsbeispielen der Figuren 8 und 9 ist die Ausnehmung 9 der Figur 11 jedoch nicht so bemessen, dass sie als Koppelantenne 4 oder als Flächenantenne wirkt, insbesondere nicht als Koppelantenne 4 für elektromagnetische Strahlung im UHF- und/ oder SHF-Frequenz- oder Wellenlängenbereich. Die drahtlose Übertragung, d.h. der Empfang und die Abstrahlung, der Daten und/ oder Energie aufweisender elektromagnetischer Strahlung ist gemäß dem Ausführungsbeispiel der Figur 11 somit ausschließlich durch die metallische, lineare Antenne bewirkt.

Die Ausnehmung 9 ist wiederum zumindest teilweise mit einem elektrisch nichtleitenden Material gefüllt (so wie im Vorstehenden bereits beschrieben), in dem die metallische, lineare Antenne aufgenommen oder eingebettet ist. Der Tray 6, insbesondere der Boden 12 und die Seitenwände 13, können aus Metall, Kunststoff, Glas oder Keramik gefertigt sein.

Die in der Figur 11 als (geometrisch) lineare Antenne ausgebildete Koppelantenne 4, umfasst einen elektrischen Leiter 26, insbesondere in Form eines metallischen Drahts oder eines metallischen Stabs. Die (geometrisch) lineare Antenne ist insbesondere als Stabantenne oder als Drahtantenne oder als Dipolantenne ausgebildet. Vorzugsweise ist ein elektrisches Bauteil 27 mit dem elektrischen Leiter 26 verbunden. Das elektrische Bauteil 27 umfasst zum Beispiel einen Signalverstärker, um die Übertragung der Daten und/ oder Energie aufweisenden elektromagnetischen Strahlung zu unterstützen, und/ oder ein Speicherelement, das insbesondere einen Identifizierungscode des Trägerelements 1 oder einen Code zur Identifizierung des Trägerelements 1 über eine externe Datenbank speichert.

Das in Figur 11 dargestellte und im Vorstehenden beschriebene Ausführungsbeispiel ist in entsprechender Weise auf eine Kassette 7 übertragbar.

Der in der Figur 10 dargestellte Tray 6 weist keine Koppelantenne 4 auf, insbesondere keine Koppelantenne 4 für elektromagnetische Strahlung im UHF- und/ oder SHF-Frequenz- oder Wellenlängenbereich. Der Tray 6 ist vorzugsweise zur Verwendung mit einem im Vorstehenden beschriebenen Trägerelement 1, 6, 7 mit einer Koppelantenne 4 vorgesehen, insbesondere zur gemeinsamen Verwendung in einem Reinigungs- oder Pflegegerät 50, wobei der Tray 6 derart ausgebildet ist, dass er eine drahtlosen Übertragung von Daten und/ oder Energie aufweisender elektromagnetischer Strahlung, bevorzugt im UHF- und/ oder SHF-Frequenz- oder Wellenlängenbereich, insbesondere zwischen einer ersten Sende- und Empfangseinheit 3 und einer zweiten Sende- und Empfangseinheit 5, besonders bevorzugt mit Hilfe einer Koppelantenne 4, möglichst wenig (negativ) beeinflusst.

Dazu ist zumindest ein Teilbereich des Bodens 12, vorzugsweise der gesamte Boden 12 aus einem elektrisch nichtleitenden Material gebildet, zum Beispiel aus Kunststoff oder Glas oder Keramik. Vorzugsweise ist das elektrisch nichtleitenden Material als Platte ausgebildet, die insbesondere in eine Ausnehmung 29 des Trays 6 eingefügt ist. Die Ausnehmung 29 wird zum Beispiel durch die Seitenwände 13 und/ oder Teilbereiche des Bodens 12 gebildet. Die Seitenwände 13 und/ oder Teilbereiche des Bodens 12 können aus Metall oder alternativ auch aus einem der genannten elektrisch nichtleitenden Materialien gebildet sein.

Das in der Figur 12 dargestellte Reinigungs- oder Pflegegerät 50 für zumindest ein medizinisches oder dentales Instrument 2 ist als Sterilisator, insbesondere als Dampfsterilisator ausgebildet. Das Reinigungs- oder Pflegegerät 50 ist zur Verwendung mit einem Trägerelement 1 mit zumindest einer Koppelantenne 4 und/ oder dem Tray der Figur 10 vorgesehen.

Der Sterilisator 50 umfasst ein Außengehäuse 51 mit mehreren Wänden und einen Deckel 52, der relativ zu den Wänden beweglich ist. An einer der Wände des Gehäuses 51 ist eine Öffnung 53 vorgesehen, die mit einem Reinigungs- oder Pflegeraum 54 des Sterilisators 50 verbunden ist. Durch diese Öffnung 53 kann ein zu reinigendes oder zu pflegendes Instrument 2 und ein Trägerelement 1, 6, 7 in den Reinigungs- oder Pflegeraum 5 eingebracht oder daraus entnommen werden.

Am Gehäuse 51 sind ein oder mehrere Bedienelemente 55 vorgesehen, die zum Beispiel zur Auswahl unterschiedlicher Betriebsprogramme oder zur Einstellung von Betriebsparametern dienen. Eine Anzeige 56 zeigt die ausgewählten Betriebsprogramme oder Betriebsparameter oder sonstige für den Betrieb des Sterilisators 50 relevante Daten oder Kennwerte an.

Im Inneren des Gehäuses 51 sind neben dem als Druckkessel ausgeführten Reinigungs- oder Pflegeraum 54 eine Versorgungsvorrichtung zum Einbringen zumindest eines Reinigungs- oder Pflegemediums, insbesondere Dampf, in den Reinigungs- oder Pflegeraum 54 und eine Steuervorrichtung 57 vorgesehen. Der Reinigungs- oder Pflegeraum 5 ist ausgebildet, das zumindest eine Trägerelement 1 aufzunehmen. Wie im Vorstehenden bereits beschrieben ist es auch möglich, dass die zweite Sende- und Empfangseinheit 5, insbesondere ein Lesegerät, an dem Sterilisator 50, insbesondere am Gehäuse 51 oder im Inneren des Gehäuses 51 angeordnet ist.

Die Steuervorrichtung 57 ist ausgebildet, den Betrieb des Reinigungs- oder Pflegegeräts 50, insbesondere den Ablauf der Reinigungs- und / oder Pflegeverfahren und die Einhaltung der Kenngrößen während der Reinigungs- und / oder Pflegeverfahren zu steuern und / oder zu regeln. Die zweite Sende- und Empfangseinheit 5 ist entweder als integraler Teil der Steuervorrichtung 57 ausgebildet und/ oder ist mit der Steuervorrichtung 57 zumindest kommunikativ verbunden. Die eine oder mehreren Antennen der zweite Sende- und Empfangseinheit 5 sind an dem Außengehäuse 51 angeordnet, zum Beispiel an einer der Wände des Außengehäuses 51, insbesondere ist zumindest eine Antenne an der Wand mit der Öffnung 53 angeordnet, und/ oder am Deckel 52 und/ oder an einem eine Wand des Außengehäuses 51, insbesondere jene Wand mit der Öffnung 53, überragende Vorsprung 58.

Die zweite Sende- und Empfangseinheit 5, insbesondere das Lesegerät, sind ausgebildet, kommunikativ mit der ersten Sende- und Empfangseinheit 3, insbesondere dem RFID-Etikett, in Verbindung zu treten, gegebenenfalls Energie mit Hilfe der Koppelantenne 4 des Trägerelements 1 auf die erste Sende- und Empfangseinheit 3 zu übertragen und die auf der ersten Sende- und Empfangseinheit 3 gespeicherten Daten mit Hilfe der Koppelantenne 4 des Trägerelements 1 drahtlos abzufragen. Die zweite Sende- und Empfangseinheit 5 kann bevorzugt weitere, insbesondere elektrische, Bauteile, zum Beispiel einen Signalverstärker für die übertragbaren Daten, eine Signalverarbeitungseinheit, eine Energiequelle oder einen Anschluss an eine Energiequelle aufweisen. Alternativ sind eines oder mehrere dieser Elemente Teil der Steuervorrichtung 57.

Bevorzugt ist die Steuervorrichtung 57 ausgebildet, die von der zweiten Sende- und Empfangseinheit 5 ausgelesen Daten zu empfangen und basierend auf diesen Daten das zumindest eine auf dem Trägerelement 1 befindliche medizinische oder dentale Instrument 2 zu identifizieren. Die Steuervorrichtung 57 kann des Weiteren auch ausgebildet sein, auf Basis der empfangenen Daten die Reinigungs- oder Pflegevorrichtung 50 zu betreiben, insbesondere ein Reinigungs- oder Pflegeverfahren auszuwählen oder Parameter für den Betrieb der Reinigungs- oder Pflegevorrichtung 50 automatisch einzustellen.

Die Steuervorrichtung 57 ist bevorzugt ausgebildet, Daten über ein von der Reinigungs- oder Pflegevorrichtung 50 durchgeführtes Reinigungs- oder Pflegeverfahren an die zweiten Sende- und Empfangseinheit 5 zu übertragen, so dass die zweiten Sende- und Empfangseinheit 5 diese Daten über die Koppelantenne 4 auf die erste Sende- und Empfangseinheit 3 überträgt, insbesondere auf das RFID-Etikett des Instruments 2. Auf die erste Sende- und Empfangseinheit 3 übertragene Daten umfassen insbesondere Angaben, ob das Instrument 2 gereinigt oder gepflegt wurde, mit welchem Verfahren es gereinigt oder gepflegt wurde, bei welchen Parametern (Temperatur, Druck, etc.) es gereinigt oder gepflegt wurde oder wie oft es in Summe bereits gereinigt oder gepflegt wurde.

Die beschriebenen oder dargestellten Ausführungsbeispiele dienen insbesondere der Veranschaulichung der Erfindung. Die in einem Ausführungsbeispiel offenbarten Merkmale sind daher nicht auf dieses Ausführungsbeispiel beschränkt, sondern sind einzeln oder gemeinsam mit einem oder mehreren Merkmalen eines der anderen Ausführungsbeispiele kombinierbar. Insbesondere sind die Art, Anzahl, Form und Position der in den einzelnen Ausführungsbeispielen dargestellten Koppelantennen beispielhaft und zwischen den Ausführungsbeispielen übertragbar.

## Patentansprüche

1. Trägerelement (1) zum Lagern zumindest eines medizinischen oder dentalen Instruments (2) in einem Reinigungs- oder Pflegegerät (50), um das zumindest eine medizinische oder dentale Instrument (2) in dem Reinigungs- oder Pflegegerät (50) zu reinigen oder zu pflegen, wobei an dem medizinischen oder dentalen Instrument (2) eine erste Sende- und Empfangseinheit (3) angeordnet ist, die zur drahtlosen Übertragung von Daten und/ oder Energie aufweisender elektromagnetischer Strahlung mit einer zweiten Sende- und Empfangseinheit (5) ausgebildet ist, wobei an dem Trägerelement (1) zumindest eine Koppelantenne (4) vorgesehen ist, die ausgebildet ist, die drahtlos zwischen der ersten Sende- und Empfangseinheit (3) und der zweiten Sende- und Empfangseinheit (5) übertragene elektromagnetische Strahlung zu empfangen und wiederum abzustrahlen, um die drahtlose Übertragung der Daten und/ oder Energie aufweisenden elektromagnetischen Strahlung zwischen der ersten Sende- und Empfangseinheit (3) und der zweiten Sende- und Empfangseinheit (5) zu unterstützen, **dadurch gekennzeichnet, dass** die zumindest eine Koppelantenne (4) eine Flächenantenne aufweist, die durch eine Öffnung oder einen Durchbruch oder einen Schlitz (11) in einer Außenwand (10; 12; 15, 16, 17) des Trägerelements (1) oder durch einen Schlitz in einer in das Trägerelement (1) eingelegten Platte gebildet ist.

2. Trägerelement (1) nach Anspruch 1, **dadurch gekennzeichnet, dass** das Trägerelement (1) eine Trägerplatte, ein Tray (6), einen Korb oder eine Kassette (7) umfasst.

3. Trägerelement (1) nach Anspruch 1 oder 2, **dadurch gekennzeichnet, dass** an dem Trägerelement (1) zumindest ein Positionierelement (8), insbesondere eine Halterung (8A), vorgesehen ist, um das zumindest eine medizinische oder dentale Instrument (2) an dem Trägerelement (1) zu positionieren, wobei das Positionierelement (8) derart ausgebildet, dass das medizinische oder dentale Instrument (2) in einer vorbestimmten Position, Orientierung und/ oder Anordnung in Bezug auf die zumindest eine Koppelantenne (4) anordenbar ist, in der die erste Sende- und Empfangseinheit (3) der zumindest einen Koppelantenne (4) zugewandt ist und/ oder sich in Richtung der zumindest einen Koppelantenne (4) erstreckt.

4. Trägerelement (1) nach einem der Ansprüche 1 - 3, **dadurch gekennzeichnet, dass** die Flächenantenne, insbesondere der Schlitz (11), von einem metallischen Bauteil des Trägerelements (1) umgeben ist.

5. Trägerelement (1) nach Anspruch 4, **dadurch gekennzeichnet, dass** die Flächenantenne, insbesondere die Öffnung oder der Durchbruch oder der Schlitz (11), mit einem elektrisch nichtleitenden Material gefüllt ist.

6. Trägerelement (1) nach einem der Ansprüche 1 - 5, **dadurch gekennzeichnet, dass** die zumindest eine Koppelantenne (4) ausgebildet ist, eine Änderung der Abstrahlrichtung der Daten und/ oder Energie aufweisenden elektromagnetischen Strahlung und/ oder eine Erhöhung der Energiedichte der Daten und/ oder Energie aufweisenden elektromagnetischen Strahlung und/ oder eine Fokussierung der Daten und/ oder Energie aufweisenden elektromagnetischen Strahlung zu bewirken.

7. Trägerelement (1) nach einem der Ansprüche 1 - 6, **dadurch gekennzeichnet, dass** die Länge der Koppelantenne (4), insbesondere der Öffnung, des Durchbruchs oder Schlitzes (11), auf die Wellenlänge oder Frequenz der zwischen der ersten und zweiten Sende- und Empfangseinheit (3, 5) zu übertragenden, Daten und/ oder Energie aufweisenden, elektromagnetischen Strahlung abgestimmt ist, insbesondere auf eine elektromagnetische Strahlung im UHF- und/ oder SHF-Frequenz- oder Wellenlängenbereich, so dass eine Länge der Koppelantenne (4), insbesondere der Öffnung, des Durchbruchs oder Schlitzes (11), vorzugsweise zumindest etwa 6,25 cm beträgt.

8. Trägerelement (1) nach einem der Ansprüche 1 - 7, **gekennzeichnet durch** eine weitere Koppelantenne (4), die eine lineare Antenne (26) aufweist, wobei die lineare Antenne (26) in der Öffnung oder dem Durchbruch oder Schlitz (11) der Flächenantenne angeordnet ist.

9. Trägerelement (1) nach Anspruch 8, **dadurch gekennzeichnet, dass** die lineare Antenne (26) als Stabantenne oder als Drahtantenne oder als Dipolantenne ausgebildet ist.

10. Trägerelement (1) nach einem der vorstehenden Ansprüche 1 - 9, **gekennzeichnet durch**
mehrere Koppelantennen (4), wobei zumindest zwei dieser mehreren Koppelantennen (4) zur Übertragung elektromagnetischer Strahlung unterschiedlicher Frequenzen ausgebildet sind.

11. Reinigungs- oder Pflegegerät (50) für zumindest ein medizinisches oder dentales Instrument (2), **gekennzeichnet durch**
ein Trägerelement (1) nach einem der vorstehenden Ansprüche und eine zweite Sende- und Empfangseinheit (5).

12. Verwendung eines Trägerelements (1) nach einem der vorstehenden Ansprüche 1 - 10 bei der Reinigung oder Pflege zumindest eines medizinischen oder dentalen Instruments (2) in einem Reinigungs- oder Pflegegerät zur Unterstützung einer drahtlosen Übertragung von Daten und/ oder Energie aufweisender elektromagnetischer Strahlung zwischen einer ersten, an dem medizinischen oder dentalen Instrument (2) angeordneten Sende- und Empfangseinheit (3) und einer zweiten Sende- und Empfangseinheit (5).

13. Verfahren zur drahtlosen Übertragung von Daten und/ oder Energie aufweisender elektromagnetischer Strahlung zwischen einer ersten, an einem medizinischen oder dentalen Instrument (2) angeordneten Sende- und Empfangseinheit (3) und einer zweiten Sende- und Empfangseinheit (5), **dadurch gekennzeichnet, dass** ein Trägerelement (1) zum Lagern des medizinischen oder dentalen Instruments (2) nach einem der Ansprüche 1 - 10 zur Verfügung gestellt wird,
das Trägerelement (1) mit dem darauf gelagerten medizinischen oder dentalen Instrument (2) mit der ersten Sende- und Empfangseinheit (3) in ein Reinigungs- oder Pflegegerät (50) zum Reinigen oder Pflegen des medizinischen oder dentalen Instruments (2) eingebracht wird, und
die Daten und/ oder Energie aufweisende elektromagnetische Strahlung drahtlos mittels der zumindest einen Koppelantenne (4) zwischen der ersten Sende- und Empfangseinheit (3) und der zweiten Sende- und Empfangseinheit (5) übertragen wird.

14. Verfahren zur drahtlosen Übertragung von Daten und/ oder Energie aufweisender elektromagnetischer Strahlung nach Anspruch 13, **dadurch gekennzeichnet, dass** die Daten und/ oder Energie aufweisende elektromagnetische Strahlung vor oder nach dem Schließen einer Öffnung des Reinigungs- oder Pflegegeräts, durch welche das Trägerelement (1) mit dem darauf gelagerten, zu reinigenden oder zu pflegenden medizinischen oder dentalen Instrument (2) mit der ersten Sende- und Empfangseinheit (3) eingebracht wird, übertragen wird.

15. Verfahren zur drahtlosen Übertragung von Daten und/ oder Energie aufweisender elektromagnetischer Strahlung nach Anspruch 13 oder 14, **dadurch gekennzeichnet, dass**
das zumindest eine medizinische oder dentale Instrument (2) mittels einer Halterung (8A) derart an dem Trägerelement (1) fixiert wird, dass das medizinische oder dentale Instrument (2) in einer vorbestimmten Position, Orientierung und/ oder Anordnung in Bezug auf die zumindest eine Koppelantenne (4) angeordnet ist, in der die erste Sende- und Empfangseinheit (3) der zumindest einen Koppelantenne (4) zugewandt ist und/ oder sich in Richtung der zumindest einen Koppelantenne (4) erstreckt.

## Claims

1. Carrier element (1) for supporting at least one medical or dental instrument (2) in a cleaning or care device (50) in order to clean or care for the at least one medical or dental instrument (2) in the cleaning or care device (50), wherein a first transmitting and receiving unit (3) is arranged on the medical or dental instrument (2), which is configured for wireless transmission of electromagnetic radiation comprising data and/or energy with a second transmitting and receiving unit (5), wherein
at least one coupling antenna (4) is provided on the carrier element (1), which is configured to receive the electromagnetic radiation transmitted wirelessly between the first transmitting and receiving unit (3) and the second transmitting and receiving unit (5) and to emit it again in order to support the wireless transmission of the electromagnetic radiation comprising data and/or energy between the first transmitting and receiving unit (3) and the second transmitting and receiving unit (5), **characterized in that**
the at least one coupling antenna (4) comprises a flat top antenna which is formed by an opening or aperture or a slot (11) in an outer wall (10, 12, 15, 16, 17) of the carrier element (1) or by a slot in a plate inserted into the carrier element (1).

2. The carrier element (1) according to Claim 1, **characterized in that** the carrier element (1) comprises a carrier plate, a tray (6), a basket or a cassette (7).

3. The carrier element (1) according to Claim 1 or 2, **characterized in that** at least one positioning element (8), in particular a holder (8A), is provided on the carrier element (1) in order to position the at least one medical or dental instrument (2) on the carrier element (1), wherein the positioning element (8) is configured such that the medical or dental instrument (2) can be arranged in a predetermined position, orientation and/or arrangement with respect to the at least one coupling antenna (4), in which the first transmitting and receiving unit (3) faces the at least one coupling antenna (4) and/or extends in the direction of the at least one coupling antenna (4).

4. The carrier element (1) according to one of Claims 1 - 3, **characterized in that** the flat top antenna, in particular slot (11), is surrounded by a metallic component of the carrier element (1).

5. The carrier element (1) according to Claim 4, **characterized in that** the flat top antenna, in particular the opening or aperture or slot (11), is filled with an electrically non-conducting material.

6. The carrier element (1) according to one of Claims 1 - 5, **characterized in that** the at least one coupling antenna (4) is configured to achieve a change of the direction of emission of the electromagnetic radiation comprising data and/or energy, and/ or an increase in the energy density of the electromagnetic radiation comprising data and/or energy, and/ or a focusing of the electromagnetic radiation comprising data and/or energy.

7. The carrier element (1) according to one of Claims 1 - 6, **characterized in that** the length of the coupling antenna (4), in particular of the opening, aperture or slot (11), is matched to the wavelength or frequency of the electromagnetic radiation comprising data and/or energy to be transmitted between the first and second transmitting and receiving unit (3, 5), in particular to electromagnetic radiation in the UHF and/or SHF frequency or wavelength range, so that a length of the coupling antenna (4), in particular of the opening, aperture or slot (11), preferably amounts to at least about 6.25 cm.

8. The carrier element (1) according to one of Claims 1 - 7, **characterized by** an additional coupling antenna (4) having a linear antenna (26), wherein the linear antenna (26) is disposed in the opening, aperture or slot (11) of the flat top antenna.

9. The carrier element (1) according to Claim 8, **characterized in that** the linear antenna (26) is formed as a rod antenna or a wire antenna or a dipole antenna.

10. The carrier element (1) according to one of the above Claims 1 - 9, **characterized by** a plurality of coupling antennas (4), wherein at least two of said plurality of coupling antennas (4) are configured to transmit electromagnetic radiation of different frequencies.

11. A cleaning or care device (50) for at least one medical or dental instrument (2),
**characterized by**
a carrier element (1) according to any one of the preceding Claims and a second transmitting and receiving unit (5).

12. Use of a carrier element (1) according to any one of Claims 1 - 10 in the cleaning or care of at least one medical or dental instrument (2) in a cleaning or care device for supporting a wireless transmission of electromagnetic radiation comprising data and/or energy between a first transmitting and receiving unit (3) arranged on the medical or dental instrument (2) and a second transmitting and receiving unit (5).

13. A method for wireless transmission of electromagnetic radiation comprising data and/or energy between a first transmitting and receiving unit (3) arranged on a medical or dental instrument (2) and a second transmitting and receiving unit (5), **characterized in that**
a carrier element (1) for supporting the medical or dental instrument (2) according to any one of Claims 1 - 10 is provided,
the carrier element (1) with the medical or dental instrument (2) having the first transmitting and receiving unit (3) mounted thereon is inserted into a cleaning or care device f(50) or cleaning or care of the medical or dental instrument (2), and
the electromagnetic radiation comprising data and/or energy is transmitted wirelessly via the at least one coupling antenna (4) between the first transmitting and receiving unit (3) and the second transmitting and receiving unit (5).

14. The method for the wireless transmission of electromagnetic radiation comprising data and/or energy according to Claim 13, **characterized in that** the electromagnetic radiation comprising data and/or energy is transmitted before or after closing an opening of the cleaning or care device, through which the carrier element (1) with the medical or dental instrument (2) to be cleaned or cared for and having the first transmitting and receiving unit (3) is introduced into the cleaning or care device.

15. The method for wireless transmission of electromagnetic radiation comprising data and/or energy according to Claim 13 or 14, **characterized in that** the at least one medical or dental instrument (2) is fixed to the carrier element (1) by a holder (8A) such that the medical or dental instrument (2) is arranged in a predetermined position, orientation and/or arrangement with respect to the at least one coupling antenna (4), in which the first transmitting and receiving unit (3) faces the at least one coupling antenna (4) and/or extends in the direction of the at least one coupling antenna (4).

## Revendications

1. Élément de support (1) pour stocker au moins un instrument médical ou dentaire (2) dans un appareil de nettoyage ou d'entretien (50) pour nettoyer ou entretenir l'au moins un instrument médical ou dentaire (2) dans l'appareil de nettoyage ou d'entretien (50), dans lequel une première unité d'émission et de réception (3) est disposée au niveau de l'instrument médical ou dentaire (2), laquelle est réalisée pour la transmission sans fil d'un rayonnement électromagnétique présentant des données et/ou de l'énergie avec une deuxième unité d'émission et de réception (5), dans lequel au moins une antenne de couplage (4) est prévue sur l'élément de support (1), laquelle est réalisée pour réceptionner le rayonnement électromagnétique transmis sans fil entre la première unité d'émission et de réception (3) et la deuxième unité d'émission et de réception (5) et l'émettre à son tour pour favoriser la transmission sans fil du rayonnement électromagnétique présentant des données et/ou de l'énergie entre la première unité d'émission et de réception (3) et la deuxième unité d'émission et de réception (5), **caractérisé en ce que**
l'au moins une antenne de couplage (4) présente une antenne plate qui est formée par une ouverture ou une traversée ou une fente (11) dans une paroi extérieure (10; 12; 15, 16, 17) de l'élément de support (1) ou par une fente dans une plaque mise en place dans l'élément de support (1).

2. Élément de support (1) selon la revendication 1, **caractérisé en ce que** l'élément de support (1) comprend une plaque de support, un plateau (6), une corbeille ou une cassette (7).

3. Élément de support (1) selon la revendication 1 ou 2, **caractérisé en ce que** l'on prévoit, au niveau de l'élément de support (1), au moins un élément de positionnement (8), en particulier une fixation (8A), pour positionner l'au moins un instrument médical ou dentaire (2) au niveau de l'élément de support (1), dans lequel l'élément de positionnement (8) est réalisé de telle sorte que l'instrument médical ou dentaire (2) peut être agencé dans une position, orientation et/ou disposition prédéfinie par rapport à l'au moins une antenne de couplage (4), dans laquelle position la première unité d'émission et de réception (3) est tournée vers l'au moins une antenne de couplage (4) et/ou s'étend en direction de l'au moins une antenne de couplage (4).

4. Élément de support (1) selon l'une des revendications 1-3, **caractérisé en ce que** l'antenne plate, en particulier la fente (11), est entourée par une pièce métallique de l'élément de support (1).

5. Élément de support (1) selon la revendication 4, **caractérisé en ce que** l'antenne plate, en particulier l'ouverture ou la traversée ou la fente (11), est remplie avec un matériau non électro-conducteur.

6. Élément de support (1) selon l'une des revendications 1-5, **caractérisé en ce que** l'au moins une antenne de couplage (4) est réalisée pour provoquer une modification de la direction d'émission du rayonnement électromagnétique présentant des données et/ou de l'énergie et/ou une augmentation de la densité d'énergie du rayonnement électromagnétique présentant des données et/ou de l'énergie et/ou une focalisation du rayonnement électromagnétique présentant des données et/ou de l'énergie.

7. Élément de support (1) selon l'une des revendications 1-6, **caractérisé en ce que** la longueur de l'antenne de couplage (4), en particulier de l'ouverture, la traversée ou la fente (11), est adaptée à la longueur d'onde ou la fréquence du rayonnement électromagnétique présentant des données et/ou de l'énergie à transmettre entre la première et la deuxième unité d'émission et de réception (3, 5), en particulier à un rayonnement électromagnétique dans la plage de longueurs d'ondes de fréquence UHF et/ou SHF, de sorte qu'une longueur de l'antenne de couplage (4), en particulier de l'ouverture, la traversée ou la fente (11), est de préférence au moins de près de 6,25 cm.

8. Élément de support (1) selon l'une des revendications 1-7, **caractérisé par** une autre antenne de couplage (4) qui présente une antenne linéaire (26), dans lequel l'antenne linéaire (26) est disposée dans l'ouverture ou la traversée ou la fente (11) de l'antenne plate.

9. Élément de support (1) selon la revendication 8, **caractérisé en ce que** l'antenne linéaire (26) est réalisée en tant qu'antenne tige ou en tant qu'antenne filaire ou en tant qu'antenne dipôle.

10. Élément de support (1) selon l'une des revendications 1-9 précédentes, **caractérisé par**
plusieurs antennes de couplage (4), dans lequel au moins deux de ces plusieurs antennes de couplage (4) sont réalisées pour la transmission d'un rayonnement électromagnétique de différentes fréquences.

11. Appareil de nettoyage ou d'entretien (50) pour au moins un instrument médical ou dentaire (2), **caractérisé par**
un élément de support (1) selon l'une des revendications précédentes et une deuxième unité d'émission et de réception (5).

12. Utilisation d'un élément de support (1) selon l'une des revendications 1-10 lors du nettoyage ou de l'entretien d'au moins un instrument médical ou dentaire (2) dans un appareil de nettoyage ou d'entretien pour favoriser une transmission sans fil d'un rayonnement électromagnétique présentant des données et/ou de l'énergie entre une première unité d'émission et de réception (3) disposée au niveau de l'instrument médical ou dentaire (2) et une deuxième unité d'émission et de réception (5).

13. Procédé pour la transmission sans fil d'un rayonnement électromagnétique présentant des données et/ou de l'énergie entre une première unité d'émission et de réception (3) disposée au niveau d'un instrument médical ou dentaire (2) et une deuxième unité d'émission et de réception (5), **caractérisé en ce qu'**un élément de support (1) pour stocker l'instrument médical ou dentaire (2) selon l'une des revendications 1-10 est fourni,
que l'élément de support (1) avec l'instrument médical ou dentaire (2) stocké sur celui-ci est introduit avec la première unité d'émission et de réception (3) dans un appareil de nettoyage ou d'entretien (50) pour le nettoyage ou l'entretien de l'instrument médical ou dentaire (2), et
que le rayonnement électromagnétique présentant des données et/ou de l'énergie est transmis sans fil au moyen de l'au moins une antenne de couplage (4) entre la première unité d'émission et de réception (3) et la deuxième unité d'émission et de réception (5).

14. Procédé pour la transmission sans fil d'un rayonnement électromagnétique présentant des données et/ou de l'énergie selon la revendication 13, **caractérisé en ce que**
le rayonnement électromagnétique présentant des données et/ou de l'énergie est transmis avant ou après la fermeture d'une ouverture de l'appareil de nettoyage ou d'entretien à travers laquelle l'élément de support (1) avec l'instrument médical ou dentaire (2) à nettoyer ou entretenir qui y est stocké dessus est introduit avec la première unité d'émission et de réception (3).

15. Procédé pour la transmission sans fil d'un rayonnement électromagnétique présentant des données et/ou de l'énergie selon la revendication 13 ou 14, **caractérisé en ce**
**qu'**au moins un instrument médical ou dentaire (2) est fixé au moyen d'une fixation (8A) à l'élément de support (1) de telle sorte que l'instrument médical ou dentaire (2) est agencé dans une position, orientation et/ou disposition prédéfinie par rapport à l'au moins une antenne de couplage (4), dans laquelle position la première unité d'émission et de réception (3) est tournée vers l'au moins une antenne de couplage (4) et/ou s'étend en direction de l'au moins une antenne de couplage (4).
